# EUROPEAN PATENT APPLICATION

(11) **EP 4 660 297 A1**
(43) Date of publication of application: **10.12.2025**
(21) Application number: 24750211.5
(22) Date of filing: 29.01.2024
(51) Int. Cl.: C12N 5/0783, A61K 35/17, A61K 39/00, A61P 37/02, A61P 37/06, A61P 37/08, C07K 16/24, C07K 16/28

(54) **METHOD FOR SELECTIVELY INDUCING ANTIGEN-SPECIFIC INDUCTIVE REGULATORY T CELLS**

(30) Priority: 30.01.2023 JP 2023012172
(71) Applicant: Juntendo Educational Foundation, Tokyo 113-8421 (JP); JUNTEN BIO Co., Ltd., Tokyo 100-0004 (JP)
(72) Inventor: UCHIDA, Koichiro, Tokyo 113-8421 (JP); TAKEDA, Kazuyoshi, Tokyo 113-8421 (JP); YOGO, Kyoko, Tokyo 100-0004 (JP)
(74) Representative: Engelhard, Markus
(86) International application number: PCT/JP2024/002648
(87) International publication number: WO 2024/162262

(57) **Abstract**

The present disclosure provides a method for selectively inducing antigen-specific inductive regulatory T cells from cells derived from a subject. Specifically, in one embodiment, the present disclosure provides a method for selectively inducing antigen-specific inductive regulatory T cells from cells derived from a subject. The method includes a step for mixing: a factor with respect to selectively inducing antigen-specific inductive regulatory T cells; cells derived from the subject; and an antigen derived from the subject or an antigen not derived from the subject, or a substance containing the antigen not derived from the subject.

## Description

### [Technical Field]

Liver transplantation has been widely used as a definitive treatment for patients with end-stage liver failure. There are 20,000 cases abroad and over 500 in Japan per year.

Transplantation is one of main treatment options for end-stage kidney, heart, liver, and pancreas failure, and despite significant advances in treatment of transplant rejection in recent years, the majority of transplants are ultimately rejected. Current immunosuppressive regimens that rely on continuous drug therapy make organ transplant recipients more susceptible to infections and cancers since even a drug cannot inhibit a response clearly directed against a transplant.

One technique to induce immune tolerance is induction of antigen-specific immunological unresponsiveness (anergy) in T cells. There is a treatment method using donor-specific Tregs obtained by co-culturing regulatory T cells (Tregs) with donor cells in the presence of IL-2 and then dividing and proliferating the Tregs, or a treatment method using Tregs obtained by administering a low dose of IL-2 to proliferate Tregs in vivo. However, these methods have problems, including a risk of activation of other T cells by IL-2 and proliferation of polyclonal Tregs that are not donor-specific.

### [Summary of Invention]

### [Solution to Problem]

In order to solve the above problem, the present inventors have newly found that bringing a specific factor into contact with a cell selectively induces an antigen-specific induced suppressor T cell. Furthermore, the present inventors found that it is important to regulate production or a function of IL-2 in induction of an antigen-specific induced suppressor T cell, and also found a new method of inducing an antigen-specific induced suppressor T cell by regulating production or a function of IL-2.

Therefore, the invention in the present application provides the following items, for example.
(Item 1) A method for selectively inducing an antigen-specific induced suppressor T cell from a cell derived from a subject, the method including
   mixing a factor selectively inducing an antigen-specific induced suppressor T cell, a cell derived from the subject, and an antigen derived from the subject or an antigen not derived from the subject or one containing the antigen not derived from the subject. (Item 2) The method according to the above item, in which the factor selectively inducing the antigen-specific induced suppressor T cell is a regulatory factor capable of regulating production of IL-2 or a regulatory factor capable of regulating a function of IL-2 produced. (Item 3) The method according to any one of the above items, in which the factor selectively inducing the antigen-specific induced suppressor T cell is an inhibitory factor capable of inhibiting production of IL-2 or an inhibitory factor capable of inhibiting a function of IL-2 produced.
(Item 4) The method according to any one of the above items, in which the factor selectively inducing the antigen-specific induced suppressor T cell is an inhibitory factor capable of inhibiting interaction of IL-2 with an IL-2 receptor (IL-2R) and/or an inhibitory factor capable of inhibiting interaction of CD80 and/or CD86 with CD28.
(Item 5) The method according to any one of the above items, in which the factor selectively inducing the antigen-specific induced suppressor T cell is the inhibitory factor capable of inhibiting interaction of IL-2 with the IL-2R, the inhibitory factor being selected from an anti-IL-2 antibody, an anti-IL-2R antibody, an anti-IL-2Rα (CD25) antibody, an anti-IL-2Rβ (CD122) antibody, or an antigen-binding fragment thereof.
(Item 6) The method according to any one of the above items, in which the factor selectively inducing the antigen-specific induced suppressor T cell is the inhibitory factor capable of inhibiting interaction of CD80 and/or CD86 with CD28, the inhibitory factor being selected from the group consisting of an anti-CD80 antibody, an anti-CD86 antibody, a bispecific antibody against CD80 and CD86, an anti-CD28 antibody, or an antigen-binding fragment thereof, a CTLA4-Ig fusion protein, and a CD28-Ig fusion protein.
(Item 7) A method for producing an induced suppressor T cell from a cell derived from a subject, the method including
   mixing a regulatory factor capable of regulating production of IL-2 or a regulatory factor capable of regulating a function of IL-2 produced, a cell derived from the subject, and an antigen derived from the subject or an antigen not derived from the subject or one containing the antigen.
(Item 8) The method according to any one of the above items, in which the regulatory factor capable of regulating production of IL-2 is selected from an inhibitory factor capable of inhibiting interaction of CD80 and/or CD86 with CD28, and a small molecular compound inhibiting production of IL-2.
(Item 9) The method according to any one of the above items, in which the regulatory factor capable of regulating the function of IL-2 produced is selected from an inhibitory factor capable of inhibiting interaction of IL-2 with an IL-2 receptor (IL-2R) and a small molecular compound inhibiting an action of IL-2.
(Item 10) The method according to any one of the above items, in which the inhibitory factor capable of inhibiting interaction of CD80 and/or CD86 with CD28 is selected from the group consisting of an anti-CD80 antibody, an anti-CD86 antibody, a bispecific antibody against CD80 and CD86, an anti-CD28 antibody, or an antigen-binding fragment thereof, a CTLA4-Ig fusion protein, and a CD28-Ig fusion protein.
(Item 11) The method according to any one of the above items, in which the inhibitory factor capable of inhibiting interaction of IL-2 with the IL-2 receptor (IL-2R) is selected from an anti-IL-2 antibody, an anti-IL-2R antibody, an anti-IL-2Rα (CD25) antibody, an anti-IL-2Rβ (CD122) antibody, or an antigen-binding fragment thereof.
(Item 12) A composition for selectively inducing an antigen-specific induced suppressor T cell from a cell derived from a subject, the composition including
   a regulatory factor capable of regulating production of IL-2 or a regulatory factor capable of regulating a function of IL-2 produced,
   the composition being brought into contact with a cell derived from the subject and an antigen derived from the subject or an antigen not derived from the subject or one containing the antigen.
(Item 12A) A regulatory factor capable of regulating production of IL-2 or a regulatory factor capable of regulating a function of IL-2 produced for selectively inducing an antigen-specific induced suppressor T cell from a cell derived from a subject, the regulatory factor being brought into contact with a cell derived from the subject and an antigen derived from the subject or an antigen not derived from the subject or one containing the antigen.
(Item 13) A composition for treating a disease of a subject mediated by an immune response, the composition including
   an antigen-specific induced suppressor T cell selectively induced by mixing a regulatory factor capable of regulating production of IL-2 or a regulatory factor capable of regulating a function of IL-2 produced, a cell derived from the subject, and an antigen derived from the subject or an antigen not derived from the subject or one containing the antigen.
(Item 13A) A method for treating a disease of a subject mediated by an immune response, the method including:
   mixing a regulatory factor capable of regulating production of IL-2 or a regulatory factor capable of regulating a function of IL-2 produced, a cell derived from the subject, and an antigen derived from the subject or an antigen not derived from the subject or one containing the antigen; and
   administering to the subject an antigen-specific induced suppressor T cell selectively induced by the mixing.
(Item 13B) An antigen-specific induced suppressor T cell for treating a disease of a subject mediated by an immune response, the antigen-specific induced suppressor T cell being selectively induced by mixing a regulatory factor capable of regulating production of IL-2 or a regulatory factor capable of regulating a function of IL-2 produced, a cell derived from the subject, and an antigen derived from the subject or an antigen not derived from the subject or one containing the antigen.
(Item 13C) Use of an antigen-specific induced suppressor T cell in production of a medicament for treating a disease of a subject mediated by an immune response, the antigen-specific induced suppressor T cell being selectively induced by mixing a regulatory factor capable of regulating production of IL-2 or a regulatory factor capable of regulating a function of IL-2 produced, a cell derived from the subject, and an antigen derived from the subject or an antigen not derived from the subject or one containing the antigen.
(Item 14) The composition according to any one of the above items, the method according to any one of the above items, the antigen-specific induced suppressor T cell according to any one of the above items, or the use according to any one of the above items, in which the disease is selected from the group consisting of transplant immune rejection, an allergy, an autoimmune disease, a graft-versus-host disease, and immune rejection caused by transplantation of an iPS cell, or an ES cell, or a cell, tissue, or organ differentiated therefrom.
(Item 15) The composition, the method, the antigen-specific induced suppressor T cell, or the use according to any one of the above items, in which the subject is a human.

In the present disclosure, it is intended that one or more of the above features may be provided in further combinations in addition to the explicit combinations. Those skilled in the art will recognize further embodiments and advantages of the present disclosure upon reading and understanding the following detailed description, if necessary.

### [Advantageous Effects of Invention]

A method of the present disclosure can selectively induce an antigen-specific induced suppressor T cell by bringing a specific factor into contact with a cell.

### [Brief Description of Drawings]

[FIG. 1] FIG. 1 shows an experimental overview of Example 1;

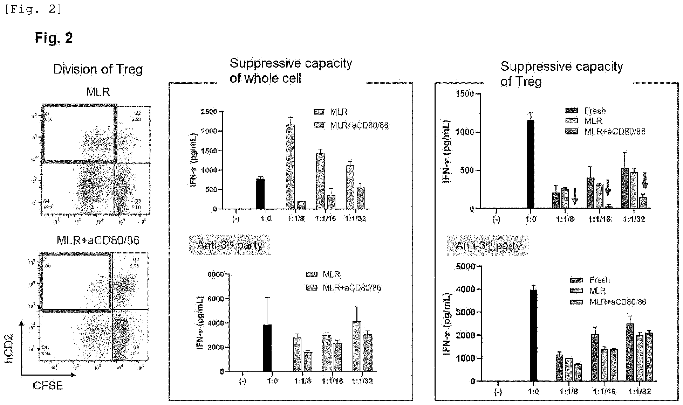

[FIG. 2] FIG. 2 shows verification of an antigen-specific suppressive capacity of whole cells and Tregs cultured in the presence of an anti-CD80/86 antibody. The left figure shows the results of verification of Treg proliferation and the right figure shows graphs of IFN-γ production by stimulation with a stimulator;
[FIG. 3] FIG. 3 shows an experimental overview of Example 1;
[FIG. 4] FIG. 4 shows verification of an antigen-specific suppressive capacity of whole cells and Tregs when IL-2 is further added to a culture in the presence of an anti-CD80/86 antibody; The left figure shows results of verification of Treg proliferation and the right figure shows graphs of IFN-γ production by stimulation with a stimulator;
[FIG. 5] FIG. 5 shows an experimental overview of Example 2;

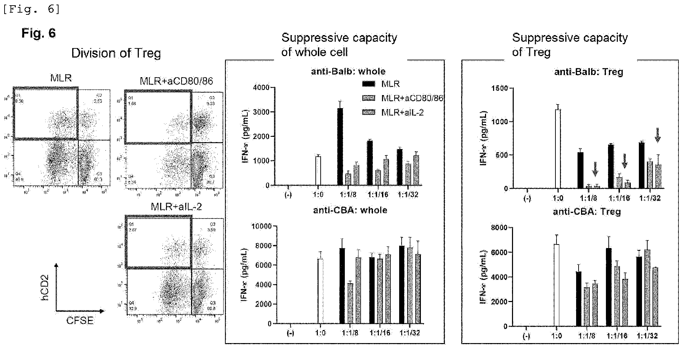

[FIG. 6] FIG. 6 shows verification of an antigen-specific suppressive capacity of whole cells and Tregs cultured in the presence of an anti-IL-2 antibody. The left figure shows the results of verification of Treg proliferation and the right figure shows graphs of IFN-γ production by stimulation with a stimulator;
[FIG. 7] FIG. 7 shows a conceptual diagram of acquisition of an antigen-specific suppressive capacity by an anti-CD80/86 antibody;
[FIG. 8] FIG. 8 shows a conceptual diagram of acquisition of an antigen-specific suppressive capacity by an anti-IL-2 antibody;
[FIG. 9] FIG. 9 shows a graph of IL-2 production by an anti-CD80/86 antibody;
[FIG. 10] FIG. 10 shows a table of IL-2 production by an anti-CD80/86 antibody;

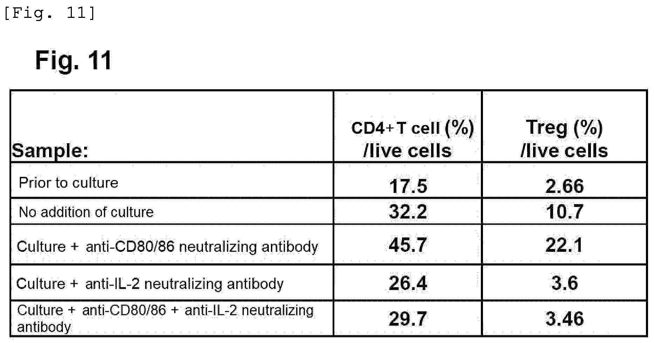

[FIG. 11]FIG. 11 shows a table of a percentage of CD4⁺ T cells in a cell population cultured in the presence of an anti-CD80/86 antibody and/or an anti-IL-2 antibody;
[FIG. 12] FIG. 12 shows verification of an antigen-specific suppressive capacity of whole cells and Tregs cultured in the presence of an anti-CD80/86 antibody and/or an anti-IL-2 antibody; and
[FIG. 13] Figure 13 shows tables summarizing the results of FIG. 12.

### [Description of Embodiments]

The present disclosure will be described with reference to the best mode thereof. It should be understood that throughout this specification, singular expressions also include the concept of their plural forms unless otherwise noted. Thus, it should be understood that singular articles (e.g., "a", "an", "the", etc. in English) also include the concept of their plural forms, unless otherwise noted. It should also be understood that terms used herein are to be used in the sense normally used in the art unless otherwise noted. Therefore, unless otherwise defined, all technical and scientific terms used herein have the same meaning as commonly understood by those skilled in the art to which the present disclosure pertains. In the event of a conflict, this specification (including definitions) shall prevail.

### (Definition of term)

As used herein, the term "about" means ±10% of the numeral value that follows.

As used herein, the phrase "immune tolerance" refers to a state in which no or suppressed specific immune responses to a specific antigen are exhibited. The immune tolerance may mean both or one of a state in which an immune cell (especially a T cell) exhibits no or suppressed specific immune responses to a specific antigen or a state in which a human exhibits no or suppressed specific immune response to a specific antigen. Induction of the immune tolerance has attracted attention because it enables a treatment of immune rejection and a treatment of an allergy. As used herein, the term "anergy" means a state in which the absence of co-stimulatory input when an antigen is presented by an antigen-presenting cell results in an inability to respond even when stimulated in the presence of a co-stimulatory the next time the antigen is presented by such a cell. Thus, as used herein, the phrase "anergy cell" refers to an immune tolerant (immune unresponsive) cell, and the phrase "anergy T cell" is an immune tolerant (immune unresponsive) T cell and also encompasses a T cell that is not activated or is unresponsive when it is again exposed to the same antigen. Note that, the phrase "PBMC (or T cell) with induced immune tolerance" and "anergic PBMC (or T cell)" are synonymous herein. It can be confirmed by, but is not limited to, confirming CD44 positivity.

As used herein, the term "subject" includes a domestic animal (e.g., a cow, sheep, a cat, a dog, a horse), a primate (e.g., a human, and a non-human primate such as a monkey), a rabbit, and a rodent (e.g., a mouse and a rat). In a specific embodiment, the subject is a human.

As used herein the term "factor" may be any substance or another element (e.g., an energy such as light, radioactivity, heat, or electricity) as long as the intended purpose can be achieved. Examples of such a substance include, but are not limited to, a protein, a polypeptide, an oligopeptide, a peptide, a polynucleotide, an oligonucleotide, a nucleotide, a nucleic acid (including, for example, DNA such as cDNA and genomic DNA, RNA such as mRNA), a polysaccharide, an oligosaccharide, a lipid, an organic small molecule (e.g., a hormone, a ligand, a transmitter, another organic small molecular compound, a molecule synthesized in combinatorial chemistry, a small molecule that can be used as a medicament (e.g., a small molecular ligand), and a complex molecule thereof.

As used herein, the phrase "regulatory factor" refers to any type of factor such as a small molecule, a protein, a nucleic acid, a lipid, a sugar, or the like that positively or negatively regulates a predetermined action (e.g., interaction, signal transduction, protein production, protein function, etc.). Thus, regulation includes induction or prevention of a change to any level such as enhancing (increasing) as well as decreasing (inhibiting, suppressing) and loss, and the phrase "regulating factor" as used herein includes "inhibitory factor", "enhancing factor" and the like. Without wishing to be bound by any particular theory, regulation of production of IL-2 or regulation of a function of IL-2 produced promotes proliferation of an induced suppressor T cell that is specific for a specific antigen. Since IL-2 also promotes proliferation of an induced suppressor T cell that is not specific for a specific antigen, it is possible to suppress proliferation of such a non-specific induced suppressor T cell by regulating production of IL-2 or regulating a function of IL-2 produced. A predetermined amount of IL-2 is preferably present because complete inhibition of production and a function of the IL-2 also suppresses proliferation of an antigen-specific induced suppressor T cell. The method, factor, and composition of the present disclosure can be adjusted to achieve the presence of the predetermined amount of IL-2.

Thus, as used herein, the term "inhibitory factor" refers to any type of factor such as a small molecule, a protein, a nucleic acid, a lipid, a sugar, or the like that can inhibit a predetermined action (e.g., interaction, signal transduction, protein production, protein function, etc.). Without wishing to be bound by any particular theory, in the present disclosure, an anergy is induced in a T cell by blocking interaction of CD80 and/or CD86 on a cell surface with CD28 to thereby inhibit a CD28 co-stimulatory signal. In the present disclosure, an inhibitory factor to be used to block the interaction of CD80 and/or CD86 with CD28 is selected from the group consisting of a small molecule, a protein, a nucleic acid, a lipid, a sugar, and a combination thereof. In one aspect, the protein is an antibody or an engineered antibody, or a cell surface molecule or an engineered cell surface molecule. In another aspect, the engineered antibody is an antigen-binding fragment. In another aspect, the engineered cell surface molecule is a fusion protein. In another aspect, the inhibitory factor is selected from the group consisting of an anti-CD80 antibody, an anti-CD86 antibody, a bispecific antibody against CD80 and CD86, an anti-CD28 antibody, or an antigen-binding fragment thereof, a CTLA4-Ig fusion protein, a CD28-Ig fusion protein. In another aspect, the CTLA4-Ig fusion protein is abatacept or belatacept. It is also envisioned that a factor that indirectly inhibits the interaction (e.g., inhibitory factor of a signal upstream or downstream signaling) may also be used in combination.

As used herein, the term "antibody" in a broad sense refers to a molecule or a population of thereof capable of specifically binding to a specific epitope on an antigen. The "antibody" in a broad sense herein may also be a full-length antibody (i.e., an antibody with an Fc portion) or an antibody lacking an Fc portion. The antibody lacking the Fc portion only has to be capable of binding to an antigen of interest, and examples of such an antibody include, but are not limited to, a Fab antibody, a F(ab')₂ antibody, a Fab' antibody, a Fv antibody, a scFv antibody, etc. The antibody may be any type of antibody, i.e., an immunoglobulin known in the art. In an exemplary embodiment, the antibody is of an isotype IgA, IgD, IgE, IgG, or IgM class. In an exemplary embodiment, the antibody described herein includes one or a plurality of alpha, delta, epsilon, gamma, and/or mu heavy chains. In an exemplary embodiment, the antibody described herein includes one or a plurality of kappa or light chains. In an exemplary aspect, the antibody is an IgG antibody, one of four human subclasses: IgG1, IgG2, IgG3, and IgG4. Examples of the antibody contemplated for use in the present disclosure include an antibody derived from Camelidae (e.g., VHH antibody), an antibody derived from shark (e.g., single chain antibody), a peptibody, a nanobody (single domain antibody), a minibody, a multispecific antibody (e.g., bispecific antibody, diabody, triabody, tetrabody), tandem di-scFV, tandem tri-scFv), etc., which are known in the art. See, for example, Kortt et al., Biomol Eng. 2001 vol. 18, 95 to 108 (2001) and Todorovska et al., J Immunol Methods. vol. 248, 47 to 66 (2001). The antibody also includes a modified antibody or an unmodified antibody. The modified antibody may be an antibody attached to various molecules, for example, polyethylene glycol. The modified antibody can be obtained by chemically modifying an antibody using a known technique. See also SEIKAGAKU (2016), Vol. 88, No. 3, pp. 380 to 385 for an artificially produced antibody and various methods for modifying/engineering an antibody.

As used herein, the term "antibody" in a narrow sense refers to an immunoglobulin or a population thereof capable of specifically binding to a specific epitope on an antigen and its engineered product is referred to as "engineered antibody". The "antibody" in a narrow sense herein may be a full-length antibody (i.e., an antibody with an Fc portion), and "engineered antibody" herein can be an engineered antibody lacking an Fc portion. Thus, the antibody in a narrow sense herein may also be referred to as a full-length antibody, and the engineered antibody may also be referred to as an engineered full-length antibody. The engineered antibody lacking an Fc portion only has to be capable of binding to an antigen of interest, and examples of such an engineered antibody include, but are not limited to, a Fab antibody, an F(ab')₂ antibody, a Fab' antibody, an Fv antibody, an scFv antibody, etc. The engineered antibody also includes a modified antibody or an unmodified antibody. The modified antibody may be an antibody attached to various molecules, for example, polyethylene glycol. The modified antibody can be obtained by chemically modifying the antibody using a known technique.

In one embodiment of the present disclosure, the "polyclonal antibody" can be produced, for example, by administering an immunogen containing an antigen of interest to a mammal (e.g., a rat, a mouse, a rabbit, a cow, a monkey, etc.), a bird, etc. to induce production of a polyclonal antibody specific for the antigen. Administration of the immunogen may be injection of one or more immunizing agents and, if desired, an adjuvant. The adjuvant may also be used to increase immune response and may include a Freund's adjuvant (complete or incomplete), a mineral gel (e.g., aluminum hydroxide), or a surfactant (e.g., lysolecithin). An immunizing protocol is known in the art and may be implemented by any method that elicits an immune response depending on a host organism to be selected (Handbook of Protein Experiments, YODOSHA CO., LTD. (2003): 86-91).

In one embodiment of the present disclosure, a "monoclonal antibody" includes a case in which individual antibodies constituting a population are identical antibodies for a substantially single epitope, except for an antibody with a possibly naturally occurring mutation in a small amount. Alternatively, the individual antibodies constituting the population may be antibodies that are substantially identical, except for an antibody with a possibly naturally occurring mutation in a small amount. The monoclonal antibody is highly specific and differs from a typical polyclonal antibody that typically contains different antibodies for different epitopes and/or typically contains different antibodies for the same epitope. In addition to its specificity, the monoclonal antibody is useful in that it can be synthesized from hybridoma culture that is not contaminated with another immunoglobulin. The adjective "monoclonal" may be characterized by being obtained from a substantially homogeneous antibody population, but it does not imply that the antibody must be produced in any particular way. For example, the monoclonal antibody may be produced in the same manner as the hybridoma method described in "Kohler G, Milstein C., Nature. 1975 Aug 7; 256(5517): 495-497". Alternatively, the monoclonal antibody may also be produced in the same manner as the recombination method described in U.S. Patent No. 4816567. Alternatively, the monoclonal antibody may be isolated from a phage antibody library in the same manner as the technique described in "Clackson et al. Nature. 1991 Aug 15; 352(6336): 624-628" or "Marks et al. J. Mol Biol. 1991 Dec 5; 222(3): 581-597". Alternatively, it can be produced by the method described in "Handbook of Protein Experiments, YODOSHA CO., LTD. (2003): 92-96".

In one embodiment of the present disclosure, a "chimeric antibody" is, for example, an antibody in which a variable region is linked to a constant region of a heterologous antibody, which can be constructed by a genetic recombination technology. A mouse-human chimeric antibody can be produced by the method described, for example, in "Roguska et al. Proc Natl Acad Sci USA. 1994 Feb 1; 91(3): 969-973". A basic method for producing the mouse-human chimeric antibody is, for example, a method in which a mouse leader sequence and a variable region sequence present in a cloned cDNA are linked to a sequence encoding a constant region of a human antibody already present in a mammalian cell expression vector. Alternatively, the mouse leader sequence and the variable region sequence present in the cloned cDNA may be linked to the sequence encoding the constant region of the human antibody and then linked to the mammalian cell expression vector. A fragment of the constant region of the human antibody can be of an H-chain constant region of any human antibody and an L-chain constant region of any human antibody, for example, Cγ1, Cγ2, Cγ3, or Cγ4 for the human H chain and Cλ or Cκ for the L chain.

In one embodiment of the present disclosure, a "humanized antibody" is, for example, an antibody that has one or more CDRs derived from a non-human species, a framework region (FR) derived from human immunoglobulin, and a constant region derived from human immunoglobulin and that binds to a desired antigen. An antibody can be humanized using various techniques known in the art (Almagro et al., Front Biosci. 2008 Jan 1; 13: 1619-1633). Examples thereof include, CDR grafting (Ozaki et al., Blood. 1999 Jun1; 93(11): 3922-3930), re-surfacing (Roguska et al., Proc Natl Acad Sci USA. 1994 Feb 1; 91(3): 969-973), FR shuffling (Damschroder et al., Mol Immunol. 2007 Apr; 44(11): 3049-3060. Epub 2007 Jan 22), and the like. To modify (and preferably improve) antigen binding, an amino acid residue in a human FR region may be substituted with a corresponding residue from a CDR donor antibody. This FR substitution can be performed by methods well known in the art (Riechmann et al., Nature. 1988 Mar 24; 332(6162): 323-327). For example, an FR residue that is important for antigen binding may be identified by modeling interaction between a CDR and an FR residue. Alternatively, an abnormal FR residue at a specific position may be identified by sequence comparison.

In one embodiment of the present disclosure, a "human antibody" is, for example, an antibody in which a region including a variable region and a constant region of a heavy chain and a variable region and a constant region of a light chain, which constitute the antibody, is derived from a gene encoding human immunoglobulin. Examples of a major production method thereof include a transgenic mouse method for producing a human antibody and a phage display method. For the transgenic mouse method for producing a human antibody, introduction of a functional human Ig gene into a mouse in which an endogenous Ig has been knocked out causes production of human antibodies with diverse antigen-binding capacities rather than a mouse antibody. Furthermore, the mouse can be immunized to obtain a human monoclonal antibody by a conventional hybridoma method. For example, it can be produced by the method described in "Lonberg et al., Int Rev Immunol. 1995; 13(1): 65-93". The phage display method is typically a system in which a foreign gene is expressed as a fusion protein without losing phage infectivity on an N-terminal side of a coat protein (g3p, g10p, etc.) of a fibrous phage such as M13 or T7, one of E. coli viruses. For example, it can be produced by the method described in "Vaughan et al. Nat Biotechnol. 1996 Mar; 14(3): 309-314".

As used herein, the phrase "antigen-binding fragment" of an antibody refers to a fragment with any length that retains an ability of the antibody to bind to an antigen. Examples of the antigen-binding fragment include, but are not limited to, Fab, F(ab')₂, Fab', Fv, and scFv.

As used herein, the phrase "fusion protein" refers to a single protein in which two or more different proteins or fragments thereof are covalently linked, or their genes are recombinantly expressed together.

As used herein, the phrase "cell derived from a subject" refers to a cell obtained from a subject to which a composition, a medicament, a cell formulation, or a cell of the present disclosure is administered or which is a target for a method of the present disclosure, or a cell derived from the cell obtained from the subject. As used herein, the phrase "antigen derived from a subject" refers to an antigen that is produced by a subject itself and that elicits an immune response, e.g., an antigen that is produced by a subject with an autoimmune disease itself and that causes the autoimmune disease in the subject. As used herein, the phrase "antigen not derived from a subject" refers to a foreign antigen that can elicit an immune response.

As used herein, the phrase "one containing an antigen not derived from a subject" refers to any substance or aggregate thereof containing an antigen that is not derived from the subject, such as a cell, cell population, or tissue expressing the antigen not derived from the subject.

As used herein, the phrase "transplant immune rejection" means that an immune system in a subject that has undergone organ, tissue, or cell transplantation attacks and damages or destroys the transplanted organ, tissue, or cell.

As used herein, the term "allergy" refers to a state in which excessive immune response is elicited against an antigen not derived from a subject. An antigen not derived from a subject that elicits the allergy is also referred to as an allergen and examples thereof include, but are not limited to, a mite antigen, an egg white antigen, a milk antigen, a wheat antigen, a peanut antigen, a soybean antigen, a buckwheat antigen, a sesame antigen, a rice antigen, a shellfish antigen, a kiwi antigen, an apple antigen, a banana antigen, a peach antigen, a tomato antigen, a tuna antigen, a salmon antigen, a mackerel antigen, a beef antigen, a chicken antigen, a pork antigen, a cat skin debris antigen, an insect antigen, a pollen antigen, a dog akin debris antigen, a fungal antigen, a bacterial antigen, a latex, a hapten, and a metal.

As used herein, the phrase "autoimmune disease" refers to any disease in which an immune system elicits an undesired immune response against its own cell, tissue, or organ. Examples of the autoimmune disease include, but are not limited to, rheumatoid arthritis, multiple sclerosis, type 1 diabetes, an inflammatory bowel disease (e.g., Crohn's disease or ulcerative colitis), systemic lupus erythematosus, psoriasis, scleroderma, an autoimmune thyroid disease, alopecia areata, Grave's disease, Guillain-Barre syndrome, a celiac disease, Sjogren's syndrome, rheumatic fever, gastritis, autoimmune atrophic gastritis, autoimmune hepatitis, isletitis, ovaritis, orchitis, uveitis, lens-induced uveitis, myasthenia gravis, primary myxoedema, pernicious anemia, autoimmune hemolytic anemia, Addison's disease, scleroderma, Goodpasture's syndrome, nephritis (e.g., glomerulonephritis), psoriasis, pemphigus vulgaris, pemphigoid, sympathetic ophthalmitis, idiopathic thrombocytopenic purpura, idiopathic leukopenia, Wegener's granuloma, and polymyositis/dermatomyositis.

As used herein, the phrase "graft-versus-host disease" means that a transplanted organ, tissue, or cell attacks and damages or destroys a cell, tissue, or organ in a subject that has undergone transplantation by an immune response.

As used herein, the phrase "immune rejection caused by transplantation of an iPS cell, or an ES cell, or a cell, tissue, or organ differentiated therefrom" refers to immune rejection caused by an antigen that the iPS cell or the ES cell has, or an antigen that a cell, tissue, or organ differentiated from the iPS cell or the ES cell has.

As used herein, the phrase "regulatory T cell" (also referred to as Treg) refers to a T cell that regulates an immune response to an antigen. The regulatory T cell may be Foxp3-positive. The regulatory T cell may also be CD4-positive or CD8-positive. Typically, the regulatory T cell may be Foxp3-positive, CD25-positive, and CD4-positive.

As used herein, the phrase "suppressor T cell" refers to a T cell that suppresses an immune response to an antigen and/or that exhibits no or suppressed immune response to an antigen.

As used herein, the phrase "antigen-specific suppressor T cell" refers to a T cell that specifically suppresses immune response to a specific antigen and/or that exhibits no or suppressed specific immune response to a specific antigen.

As used herein, the phrase "induced suppressor T cell" means a suppressor T cell induced by stimulation with a method, composition, or factor of the present disclosure, and refers to a cell population including a regulatory T cell (e.g., a FOXP3-positive CD4-positive CD25-positive T cell) and a suppressor T cell (e.g., a CD4-positive anergy T cell or a CD8-positive anergy T cell). The CD8-positive anergy cell and the CD4-positive anergy cell are rich in a CD44-positive cell, and the CD8-positive cell and/or the CD4-positive cell may be CD44-positive. In addition to CD44, CD45RA/CD45RO can also be used to confirm production of an anergy cell. For example, the CD8-positive anergy T cell and/or the CD4-positive anergy T cell are CD45RA-negative and CD45RO-positive.

As used herein, the phrase "antigen-specific induced suppressor T cell" refers to an induced suppressor T cell that specifically suppresses immune response to a specific antigen and/or that exhibits no or suppressed specific immune response to a specific antigen.

As used herein the phrase "selectively induce an antigen-specific induced suppressor T cell" does not necessarily mean that the induced suppressor T cell is 100% antigen-specific and refers to induction of an increase in percentage of the antigen-specific induced suppressor T cell when the cell is stimulated by a method, composition, or factor of the present disclosure (e.g., when the cell is brought into contact with a factor that selectively induces the antigen-specific induced suppressor T cell) as compared to when the cell is not stimulated by a method, composition, or factor of the present disclosure (e.g., when the cell is not brought into contact with a factor that selectively induces the antigen-specific induced suppressor T cell).

As used herein, the phrase "factor that selectively induces the antigen-specific induced suppressor T cell" does not necessarily mean that the induced suppressor T cell is 100% antigen-specific and refers to any factor that induces an increase in percentage of the antigen-specific induced suppressor T cell as compared to when the factor is not brought into contact with the cell.

### (Preferred Embodiment)

While description of preferred embodiments is provided below, it should be understood that these embodiments are illustrative of the present disclosure and the scope of the present disclosure is not limited to such preferred embodiments. It should also be understood that those skilled in the art can easily make modifications, changes, etc. that are within the scope of the present disclosure with reference to the following preferred examples. Those skilled in the art may combine any embodiments as appropriate considering the description herein. It is understood that the following embodiments of the present disclosure may also be used alone or in combination.

The present inventors have found that an antigen-specific suppressive capacity is induced in an induced suppressor T cell by regulating production of IL-2, which is a growth factor, or a function of IL-2 produced. The present inventors have also found a new culture method to induce antigen specificity by adding a regulatory factor capable of regulating production of IL-2 or a regulatory factor capable of regulating a function of IL-2 produced for the purpose of suppressing non-antigen specific division and proliferation of an induced suppressor T cell.

In one aspect, the present disclosure provides a method for selectively inducing an antigen-specific induced suppressor T cell from a cell derived from a subject, the method including mixing a factor selectively inducing an antigen-specific induced suppressor T cell, a cell derived from the subject, and an antigen derived from the subject or an antigen not derived from the subject or one containing the antigen. The one containing the antigen may be a cell, which may be irradiated to prevent proliferation and activation of the cell.

In another aspect, the present disclosure provides a method for producing an induced suppressor T cell from a cell derived from a subject, the method including mixing an inhibitory factor capable of inhibiting production of IL-2 or an inhibitory factor capable of inhibiting a function of IL-2 produced, a cell derived from the subject, and an antigen derived from the subject or an antigen not derived from the subject or one containing the antigen.

In another aspect, the present disclosure provides a method for suppressing proliferation of an induced suppressor T cell that is nonspecific for a specific antigen in production of an induced suppressor T cell from a cell derived from a subject, the method including mixing a factor selectively inducing an antigen-nonspecific induced suppressor T cell, a cell derived from the subject, and an antigen derived from the subject or an antigen not derived from the subject or one containing the antigen.

In some embodiments, a method of the present disclosure includes culturing a mixture including the factor, the cell derived from the subject, and the antigen derived from the subject, or the antigen not derived from a subject, or one containing the antigen. After the culturing, an induced suppressor T cell of the present disclosure can include a CD4-positive anergy T cell, a CD8-positive T cell, and a regulatory T cell. The regulatory T cell exhibits a cell surface marker such as Foxp3⁺, preferably a cell surface marker such as CD4⁺, CD25⁺, and Foxp3⁺.

In some embodiments, a percentage of CD4⁺ T cells in a culture at the end of culturing using a method, factor, and composition of the present disclosure may be about 20% or more, about 25% or more, about 30% or more, about 35% or more, about 40% or more, about 45% or more, or about 50% or more of total cells. In a preferred embodiment, the percentage of CD4⁺ T cells in the culture may be about 35% or more of total cells.

In a further embodiment, a percentage of regulatory T cells (e.g., CD4⁺Foxp3⁺ T cells) in a culture at the end of culturing using a method, factor, and composition of the present disclosure is about 5% or more, about 10% or more, about 15% or more, about 20% or more, or about 25% or more of total cells. In a preferred embodiment, the percentage of regulatory T cells in the culture may be about 15% or more of total cells.

In some embodiments, a method, factor, and composition of the present disclosure can increase a percentage of CD4⁺ regulatory T cells (e.g., CD4⁺Foxp3⁺ T cells) in CD4⁺ T cells. The percentage of the CD4⁺ regulatory T cells (e.g., CD4⁺Foxp3⁺ T cells) in the CD4⁺ T cells in a culture at the end of culturing using the method, factor, and composition of the present disclosure may be about 30% or more, about 35% or more, about 40% or more, about 45% or more, about 50% or more, about 55% or more, about 60% or more, about 65% or more, about 70% or more, about 80% or more, about 90% or more, or about 100%. In a preferred embodiment, the percentage of the CD4⁺ regulatory T cells (e.g., CD4⁺Foxp3⁺ T cells) in the CD4⁺ T cells in the culture at the end of culturing using the method, factor, and composition of the present disclosure may be about 50% or more.

In some embodiments, the cell may be a peripheral blood mononuclear cell (PBMC), a spleen cell, a bone marrow cell, a lymph node cell, or any combination thereof.

In some embodiments, a method of the present disclosure is performed without addition of interleukin-2 (IL-2).

In some embodiments, a factor selectively inducing an antigen-specific induced suppressor T cell may be a regulatory factor capable of regulating production of IL-2 or a regulatory factor capable of regulating a function of IL-2 produced. The regulation can preferably be negative regulation. Without wishing to be bound by any particular theory, since IL-2 also promotes proliferation of an induced suppressor T cell that is not specific for a specific antigen, it is possible to suppress proliferation of such a non-specific induced suppressor T cell by regulating production of IL-2 or regulating a function of IL-2 produced. A predetermined amount of IL-2 is preferably present because complete inhibition of production and a function of the IL-2 also inhibits proliferation of an antigen-specific induced suppressor T cell.

In some embodiments, a predetermined amount of IL-2 may be about 100 pg/mL or less, about 90 pg/mL or less, about 80 pg/mL or less, about 70 pg/mL or less, about 60 pg/mL or less, about 50 pg/mL or less, about 40 pg/mL or less, about 30 pg/mL or less, about 20 pg/mL or less, about 19 pg/mL or less, about 18 pg/mL or less, about 17 pg/mL or less, about 16 pg/mL or less, about 15 pg/mL or less, about 14 pg/mL or less, about 13 pg/mL or less, about 12 pg/mL or less, about 11 pg/mL or less, about 10 pg/mL or less, about 9 pg/mL or less, about 8 pg/mL or less, about 7 pg/mL or less, about 6 pg/mL or less, or about 5 pg/mL or less in a culture. The predetermined amount is an amount of IL-2 at a cell concentration of about 0.5 to 2 x 10⁶ cells/mL, and the amount of IL-2 may vary depending on the number of cells in culture as appropriate. The predetermined amount of IL-2 may also vary depending on an origin. An appropriate predetermined amount of IL-2 can be determined as appropriate based on Examples 3 and 6.

In some embodiments, IL-2 or an anti-IL-2 antibody may be added at the start or during the course of culture as appropriate for achieving the predetermined amount of IL-2 in culture.

In some embodiments, a suppressive capacity against an antigen of an antigen-specific induced suppressor T cell or a cell population containing the cell produced by a method or factor of the present disclosure is about 30% or more (i.e. 70% or less immune response relative to the reference), about 40% or more, about 50% or more, about 60% or more, about 70% or more, about 80% or more, about 90% or more, about 95% or more, about 96% or more, about 97% or more, about 98% or more, about 99% or more, and in a preferred embodiment, the suppressive capacity against an antigen of the antigen-specific induced suppressor T cell or the cell population containing the cell produced by the method or factor of the present disclosure may be about 50% or more on the basis of an immune response by a T cell or a cell population thereof cultured without the method or factor of the present disclosure.

In some embodiments, a factor that selectively induces an antigen-specific induced suppressor T cell can be a regulatory or inhibitory factor capable of regulating or inhibiting production of IL-2, or a regulatory or inhibitory factor capable of regulating or inhibiting a function of IL-2 produced, or both thereof.

In some embodiments, a factor selectively inducing an antigen-specific induced suppressor T cell may be an inhibitory factor inhibiting interaction of IL-2 with an IL-2 receptor (IL-2R) and/or an inhibitory factor capable of inhibiting interaction of CD80 and/or CD86 with CD28.

Examples of the inhibitory factor capable of inhibiting interaction of IL-2 with the IL-2 receptor (IL-2R) include, but are not limited to, an anti-IL-2 antibody, an anti-IL-2R antibody, an anti-IL-2Rα (CD25) antibody, an anti-IL-2Rβ (CD122) antibody, or an antigen-binding fragment thereof.

Examples of the inhibitory factor capable of inhibiting production of IL-2 include an inhibitory factor capable of inhibiting interaction of CD80 and/or CD86 with CD28, or a small molecular compound inhibiting production of IL-2.

Examples of the regulatory or inhibitory factor capable of regulating or inhibiting the function of IL-2 produced include, but are not limited to, an inhibitory factor capable of inhibiting interaction of IL-2 with an IL-2 receptor (IL-2R), a small molecular compound inhibiting an action of IL-2, and the like.

Examples of the small molecular compound inhibiting production of IL-2 include, but are not limited to, a calcineurin inhibitor, a steroidal anti-inflammatory drug, and the like. Examples of the calcineurin inhibitor include tacrolimus, cyclosporine, and the like. The calcineurin dephosphorylates a nuclear factor of an activated T cell (NFAT), which allows the NFAT to enter the nucleus and bind to an interleukin-2 promoting agent. Blocking this process inhibits IL-2 production. Examples of the steroidal anti-inflammatory drug include, but are not limited to, mometasone furan carboxylate, clobetasol propionate, loteprednol etabonate, difluprednate, dexamethasone, amcinonide, flurandrenolide, prednisolone, fluocinolone acetonide, desonide, triamcinolone acetonide, budesonide, fludrocortisone acetate, fluocinonide, methylprednisolone, betamethasone, desoximetasone, halcinonide, fluorometholone, beclometasone dipropionate, and dutasteride. Examples of the small molecular compound inhibiting the action of IL-2 include rapamycin (sirolimus), SDZ RAD, and the like. The rapamycin suppresses intracellular signaling and cell proliferation, inhibiting a lymphocyte response to IL-2 to prevent activation of a T lymphocyte.

Examples of the inhibitory factor capable of inhibiting interaction of CD80 and/or CD86 with CD28 include, but are not limited to, an anti-CD80 antibody, an anti-CD86 antibody, a bispecific antibody against CD80 and CD86, an anti-CD28 antibody, or an antigen-binding fragment thereof, a CTLA4-Ig fusion protein, a CD28-Ig fusion protein, and the like. The CTLA4-Ig fusion protein can be abatacept or belatacept.

In some embodiments, CD80 and/or CD86 are expressed by an antigen-presenting cell and CD28 is expressed by a T cell. In a certain embodiment, an inhibitory factor capable of inhibiting interaction of CD80 and/or CD86 with CD28 can be an anti-CD80 and/or anti-CD86 antibody or a CTLA4-Ig fusion protein. An inhibitory factor envisaged for use in the present disclosure includes a CTLA-4 Ig fusion protein as described above. The CTLA-4 Ig fusion protein competes with CD28, a co-stimulatory receptor on a T cell, for binding to CD80/CD86 on an antigen-presenting cell and thus functions to inhibit activation of the T cell. In the present disclosure, abatacept (Orencia (registered trademark)), belatacept, or Maxy-4 are envisaged as the CTLA-4 Ig fusion protein. Belatacept contains two amino acid substitutions (L104E and A29Y) that significantly increase binding avidity to CD80 and CD86 (see Davies JK et al., Cell Transplant. (2012); 21(9): 2047 to 61, Adams AB et al., J Immunol. (2016) 197(6): 2045 to 50). Another inhibitory factor that is expected to have a similar effect to that of the CTLA4-Ig fusion protein includes a CD28-Ig fusion protein (Peach RJ et al., J Exp Med. (1994) 180(6): 2049 to 2058). The inhibitory factor of the present disclosure may also be used in a form of a nucleic acid. By way of example, it is envisaged that a nucleic acid encoding the CTLA4-Ig fusion protein is introduced into a cell via an adenovirus vector or the like and expressed therein. See, for example, Jin YZ et al., Transplant Proc. (2003); 35(8): 3156 to 9.

In a further aspect, provided is a composition for selectively inducing an antigen-specific induced suppressor T cell from a cell derived from a subject, the composition including a regulatory factor capable of regulating production of IL-2 or a regulatory factor capable of regulating a function of IL-2 produced, the composition being brought into contact with a cell derived from the subject and an antigen derived from the subject or an antigen not derived from the subject or one containing the antigen.

### (Procedure for producing autologous regulatory T cell)

A typical method for producing an autologous induced suppressor T cell will be described.

### Prior Confirmation

In one embodiment, prior confirmation can be performed as follows. Various numerical values, reagents, procedures, etc. illustrated below are representative examples, and those skilled in the art may modify them as appropriate for the prior confirmation.

A donor and a patient are subjected to a screening test for an infectious disease, and the donor should be confirmed negative for all of an HBs antigen, an HCV antibody, HIV-1/2, and an HTLV-1 antibody.

### 1. Separation of donor lymphocyte (performed under aseptic condition)

In one embodiment, a donor lymphocyte can be separated as follows. Various numerical values, reagents, procedures, etc. illustrated below are representative examples, and those skilled in the art may modify them as appropriate for the separation of the donor lymphocyte.
· A donor lymphocyte is collected by apheresis into a collection bag, which is then irradiated.
The thus-irradiated peripheral blood mononuclear cell is placed in a centrifuge tube containing an appropriate amount of Ficoll-Paque PREMIUM (GE Healthcare #17-5442-02) or Lymphocyte Separation Solution (NACALAI TESQUE, INC. #20828) (e.g., 20 mL) and centrifuged at 860 G at 22°C for 20 minutes.
· A supernatant is discarded and a cell suspension containing a lymphocyte layer is transferred to another centrifuge tube (e.g., two 50 mL centrifuge tubes).
· The centrifuge tube containing the cell suspension is added with saline (e.g., an appropriate amount to a total volume of 50 mL) and mixed well by repeated aspiration and discharge with a syringe (e.g., a 50 mL syringe with an 18G needle) or a pipette.
· The resultant is centrifuged at 500 G at 22°C for 10 minutes (an accelerator of a centrifuge may be set to FAST and a brake to FAST).
· A supernatant is discarded, saline is added again (e.g., an appropriate amount to a total volume of 50 mL), and a cell pellet is mixed well by repeated aspiration and discharge with a pipette.
· The resultant is centrifuged at 500 G at 22°C for 5 minutes (an accelerator of a centrifuge may be set to FAST and a brake to FAST).
· A supernatant is discarded.
· An ALyS505N-0 culture solution (Cell Science & Technology Institute, Inc. (CSTI) 1020P10) containing plasma collected from the donor in advance is added to a cell pellet (e.g., an appropriate amount to a total volume of 31 mL) and mixed well by repeated aspiration and discharge with a pipette.
· An appropriate amount (e.g., 0.3 mL) of the resultant is withdrawn with a syringe (e.g., a 1 mL syringe with an 18G needle) or a pipette to check a cell count and a viable cell count.

### 2. Cryopreservation of donor lymphocyte (performed under aseptic condition)

In one embodiment, a donor lymphocyte can be cryopreserved as follows. Various numerical values, reagents, procedures, etc. illustrated below are representative examples, and those skilled in the art may modify them as appropriate for cryopreservation of the donor lymphocyte.
· A freezing bag (e.g., FREEZE BAG F-050 25mL Freezing Bag NIPRO CORPORATION 89-101) is opened under an aseptic condition, and necessary information (date, serial number, and donor name) is written on a label.
· A cell suspension was taken with a syringe (e.g., a 30 mL syringe with an 18G needle) and placed in the freezing bag.
· An ACD solution (Terumo Corporation TP-A05ACD, e.g., 2 mL for 15 mL of the cell suspension) is added to the freezing bag containing the cell suspension and the bag is cooled by placing it between coolants which have been cooled at 4°C for about 10 minutes.
· For example, 8.5 mL of CP-1 (KYOKUTO PHARMACEUTICAL INDUSTRIAL CO., LTD 551-27202-4 Cell cryoprotective solution CP-1) which has been cooled at 4°C is added to the freezing bag for about 1.5 minutes using a syringe (e.g., a 20 mL syringe with a 18G needle). At this time, the freezing bag is slowly agitated.
· Air is completely removed from the freezing bag and its port using a syringe.
· The freezing bag is sealed using a tube sealer, cooled at 4°C for about 5 to 10 minutes, and then stored in a freezer at -80°C.

### 3. Thawing of donor lymphocyte (performed under aseptic condition)

In one embodiment, a donor lymphocyte can be thawed as follows. Various numerical values, reagents, procedures, etc. illustrated below are representative examples, and those skilled in the art may modify them as appropriate for thawing of the donor lymphocyte.
· The freezing bag in which the donor cell is stored is thawed in, for example, a thermostatic bath at 37°C. Subsequent procedures are preferably performed under an aseptic condition.
· The cell suspension is withdrawn from the thus-thawed freezing bag using a syringe (e.g., a 50 mL syringe with an 18G needle) and transferred to a centrifuge tube (e.g., two 50 mL centrifuge tubes with 12.5 mL each).
· For example, a 5% albumin solution (NIHON PHARMACEUTICAL CO., LTD. 123146364, a 5% donated albumin for intravenous injection 12.5 g/250 mL) (e.g., 37.5 mL for 12.5 mL of the cell suspension) is added to the centrifuge tube containing the cell suspension and mixed well. Then, the centrifuge tube is left to stand for about 5 minutes.
· The resultant is centrifuged, for example, at 600 G at 22°C for 10 minutes (e.g., preferably, an accelerator of a centrifuge is set to FAST and a brake to SLOW).
· A supernatant is gently discarded, and a cell pellet is suspended in an appropriate solution such as saline with albumin (e.g., made from 25 mL of a 5% albumin solution and 19 mL of saline) for washing.
· The resultant is centrifuged, for example, at 600 G at 22°C for 10 minutes (e.g., preferably, an accelerator of a centrifuge is set to FAST and a brake to SLOW).
· A supernatant is gently discarded and a cell pellet is suspended in an ALyS505N culture solution (e.g., 10 mL for a 50 mL centrifuge tube).
· An anti-human CD80 antibody (e.g., m2D10.4; Cat. No. 16-0809-85, eBioscience) and an anti-human CD86 antibody (e.g., IT2.2; Cat. No. 16-0869-85, eBioscience) (or an inhibitory factor such as a CTLA4-Ig fusion protein (e.g., belatacept)) are added in a culture bag (e.g., NIPRO CORPORATION 87598 Nipro Medium ALyS505NB10) containing an ALyS505N-0 culture solution or equivalent at a final concentration of 10 µg/mL, respectively, and the cell suspension is added to this culture bag by injection using a syringe (e.g., a 20 mL syringe with an 18G needle). In one example, a total fluid volume in the culture bag is approximately 840 mL.

### 4. From separation of patient lymphocyte to initiation of primary culture (under aseptic condition)

In one embodiment, a patient lymphocyte can be separated as follows. Various numerical values, reagents, procedures, etc. illustrated below are representative examples, and those skilled in the art may modify them as appropriate for separation of the patient lymphocyte.
· Plasma collected from the patient is heated in a thermostatic bath, e.g., at 56°C for 30 minutes to be deactivated. One that is not used immediately is cryopreserved.
· Peripheral blood collected from the patient is placed in a centrifuge tube containing an appropriate amount of an appropriate medium, e.g., Ficoll-Paque (e.g., 20 mL), and is centrifuged, for example, at 860 G at 22°C for 20 minutes (e.g., preferably, an accelerator of a centrifuge is set to SLOW and a brake to SLOW).
· A supernatant is discarded and a cell suspension including a lymphocyte layer is transferred to another centrifuge tube (e.g., two 50 mL centrifuge tubes).
· The centrifuge tube containing the cell suspension is added with saline (e.g., an appropriate amount to a total volume of 50 mL) and mixed well by repeated aspiration and discharge with a pipette.
· For example, the resultant is centrifuged at 500 G at 22°C for 10 minutes (an accelerator of a centrifuge may be set to FAST and a brake to FAST).
· A supernatant is discarded, saline is added again (e.g., an appropriate amount to a total volume of 50 mL), and a cell pellet is mixed well by repeated aspiration and discharge with a pipette.
· For example, the resultant is centrifuged at 500 G at 22°C for 5 minutes (an accelerator of a centrifuge may be set to FAST and a brake to FAST).
· A supernatant is discarded and a cell pellet is suspended in an ALyS505N-0 culture solution (e.g., 10 mL) to produce a cell suspension (e.g., the ALyS505N-0 culture medium is added to a total volume of 20 mL). Here, about 0.5 mL of the cell suspension is withdrawn to check a cell count, a viable cell count, and expression of a surface antigen.
· The deactivated plasma derived from the patient is added to the culture bag containing the donor cell and the inhibitory factor such as the antibody in the ALyS505N-0 culture solution prepared in "3. Thawing of donor lymphocyte".
· The cell suspension derived from the patient is added to the culture bag by injection using a syringe (e.g., a 20 mL syringe with an 18G needle), and the culture bag is sealed using a tube sealer. In one example, a total fluid volume in the culture bag is approximately 1000 mL.
· The resultant is cultured in an incubator at 37°C for one week, for example.

### 5-1. Medium exchange (e.g., Week 1, preferably under aseptic condition)

In one embodiment, medium exchange can be performed as follows. Various numerical values, reagents, procedures, etc. illustrated below are representative examples, and those skilled in the art may modify them as appropriate for medium exchange.
· The culture bag is removed from the incubator and the content thereof is dispensed into centrifuge tubes (e.g., four 225 mL centrifuge tubes).
· For example, the resultant is centrifuged at 600 G at 22°C for 10 minutes (e.g., preferably, an accelerator of a centrifuge may be set to FAST and a brake to FAST).
· A supernatant is gently discarded and a cell pellet is suspended in, for example, an ALyS505N-0 culture solution to produce a cell suspension (e.g., the ALyS505N-0 culture solution is added to a total volume of 20 mL). Here, about 0.3 mL is withdrawn from the cell suspension to check a cell count and a viable cell count.
· For example, the cell suspension is added to the culture bag containing the ALyS505N-0 culture solution by injection using a syringe (e.g., a 20 mL syringe with an 18G needle).
· An anti-human CD80 antibody (e.g., 2D10.4) dilution and an anti-human CD86 antibody (e.g., IT2.2) dilution (or an inhibitory factor such as a CTLA4-Ig fusion protein (e.g., belatacept)) are added in the culture bag at a final concentration of 10 µg/mL, respectively, by injection using a syringe (e.g., a 20 mL syringe with an 18G needle).

### 5-2. Thawing of donor lymphocyte

· From antigen restimulation to initiation of secondary culture (e.g., Week 1, under aseptic condition) In one embodiment, thawing of a donor lymphocyte,
· from antigen restimulation to secondary culture initiation can be performed as follows. Various numerical values, reagents, procedures, etc. illustrated below are representative examples, and those skilled in the art may modify them as appropriate for thawing of the donor lymphocyte,
· from antigen restimulation to initiation of secondary culture.
· The freezing bag in which the donor cell and the deactivated plasma derived from the patient are stored is thawed in, for example, a thermostatic bath at 37°C. Subsequent procedures are preferably performed under an aseptic condition.
· The cell suspension is withdrawn from the thus-thawed freezing bag using a syringe (e.g., a 50 mL syringe with an 18G needle) and transferred to a centrifuge tube (e.g., two 50 mL centrifuge tubes).
· A 5% albumin solution (e.g., about 50 mL in total for two 50 mL centrifuge tubes) is added to the centrifuge tubes containing the donor cell suspension and mixed well. Then, the centrifuge tubes are left to stand for about 5 minutes.
· The resultant is centrifuged, for example, at 600 G at 22°C for 10 minutes (an accelerator of a centrifuge is set to FAST and a brake to SLOW).
· A supernatant is gently discarded, and a cell pellet is suspended in saline with albumin (e.g., made from 25 mL of a 5% albumin solution and 19 mL of saline) for washing.
· The resultant is centrifuged, for example, at 600 G at 22°C for 10 minutes (an accelerator of a centrifuge is set to FAST and a brake to SLOW).
· A supernatant is gently discarded and a cell pellet is suspended in, for example, an ALyS505N-0 culture solution (e.g., 10 mL for a 50 mL centrifuge tube).
· The thawed deactivated plasma derived from the patient (e.g., 10 mL) is added to the culture bag containing the patient cell and the inhibitory factor such as the antibody in the ALyS505N culture solution prepared in "3. Thawing of donor lymphocyte" by injection using a syringe (e.g., 20 mL syringe with a 18G needle) and the cell suspension is also added to this culture bag by injection using a syringe (e.g., a 20 mL syringe with an 18G needle). In one example, a total fluid volume in the culture bag is approximately 1000 mL.
· The culture bag is sealed using a tube sealer.
· The resultant is cultured in an incubator at 37°C for one week, for example.

### 6. Test during secondary culture (culture cell sampling test)

In one embodiment, a test during secondary culture can be performed as follows. Various numerical values, reagents, procedures, etc. illustrated below are representative examples, and those skilled in the art may modify them as appropriate for the test during secondary culture.
· Representatively, a small amount of the culture solution is withdrawn from the culture bag on the third day after the start of secondary culture (Day 10 of the total culture period) and tested for, for example, mycoplasma contamination.

### 7. Collection and filling of cultured lymphocyte

### (performed under aseptic condition)

In one embodiment, a cultured lymphocyte can be collected and
· filled as follows. Various numerical values, reagents, procedures, etc. illustrated below are representative examples, and those skilled in the art may modify them as appropriate the collection and
· filling of the cultured lymphocyte.
For example, on the 7th day after the start of secondary culture (Day 14 of the total culture period), the culture bag is removed from the incubator and the content thereof is dispensed into centrifuge tubes (e.g., four 225 mL centrifuge tubes).
· For example, the resultant is centrifuged, for example, at 600 G at 22°C for 10 minutes (an accelerator of a centrifuge may be set to FAST and a brake to FAST).
· A supernatant is gently discarded and a cell pellet is suspended in saline.
· For example, the resultant is centrifuged, for example, at 600 G at 22°C for 10 minutes (e.g., preferably, an accelerator of a centrifuge may be set to FAST and a brake to FAST).
· A supernatant is gently discarded and a cell pellet is suspended in saline (e.g., 10 mL) to produce a cell suspension.
· The cell suspension is gently placed in a centrifuge tube (e.g., 50 mL centrifuge tube) containing an appropriate amount of Ficoll-Paque (e.g., 20 mL) and layered.
· The resultant is centrifuged, for example, at 860 G at 22°C for 20 minutes (an accelerator of a centrifuge is set to SLOW and a brake to SLOW).
· A supernatant is discarded and a cell suspension containing a lymphocyte layer is transferred to another centrifuge tube (e.g., a 50 mL centrifuge tube).
· The centrifuge tube containing the cell suspension is added with saline (e.g., an appropriate amount to a total volume of 50 mL) and mixed well by repeated aspiration and discharge with a syringe (e.g., a 50 mL syringe with an 18G needle).
· For example, the resultant is centrifuged, for example, at 500 G at 22°C for 10 minutes (an accelerator of a centrifuge may be set to FAST and a brake to FAST).
· About 5 mL of a supernatant is left and the remainder is discarded, the supernatant is mixed well by repeated aspiration and discharge with a pipette.
· Saline is added (e.g., an appropriate amount to a total volume of 50 mL) and mixed well by repeated aspiration and discharge with a syringe (e.g., a 50 mL syringe with an 18G needle) (a).
· For example, the resultant is centrifuged at 500 G at 22°C for 5 minutes (an accelerator of a centrifuge may be set to FAST and a brake to FAST) (b).
· About 5 mL of a supernatant is left and the remainder is discarded, the supernatant is mixed well by repeated aspiration and discharge with a pipette (c).
· (a), (b), and (c) are repeated twice more.
· An appropriate amount (e.g., 4 mL) is withdrawn from the supernatant after the last centrifugation and subjected to a sterility test and a mycoplasma test.
·The supernatant is suspended in saline again, and the resulting cell suspension is transferred to a final container (e.g., a 100 mL bottle for saline). An appropriate amount (e.g., 4 mL) is withdrawn and the final product is checked for a cell count, a viable cell count, expression of a surface antigen, and an endotoxin content.

### 8. Secondary packaging

In one embodiment, secondary packaging can be performed as follows. Various numerical values, reagents, procedures, etc. illustrated below are representative examples, and those skilled in the art may modify them as appropriate for secondary packaging.
· Representatively, a subject ID, serial number, and expiration date are inputted and printed on a label based on the appropriate standard (representatively, NUHCPC-M-12-ATREG), and the label is affixed to the container.
· The "PRECAUTIONS CONCERNING DOSAGE AND ADMINISTRATION, INDICATIONS, AND USE or HANDLING" is issued in accordance with the appropriate standard (representatively, NUHCPC-PMF-ATREG14).
· A test article and the "PRECAUTIONS CONCERNING DOSAGE AND ADMINISTRATION, INDICATIONS, AND USE or HANDLING" is stored in a plastic bag with a zipper.
· The plastic bag is stored in a monitoring unit in a transfer container until shipping.

In one embodiment, a cell formulation can be produced as follows. Various numerical values, etc. illustrated below are representative examples, and those skilled in the art may modify them as appropriate for producing the cell formulation.
1) About 19 days prior to administration, apheresis is performed on a donor at a medical institution, the resulting donor apheresis product is irradiated at 30 Gy to eliminate a cell proliferative ability, and then shipped to a cell culture and processing facility where a cell is processed.
2) After receipt of the donor apheresis product, a donor mononuclear cell is separated and collected by density gradient centrifugation at the cell culture and processing facility, then frozen in two portions, and stored at -80 ± 10°C.
3) About 14 days prior to administration, apheresis is performed on a recipient at a medical institution, and the resulting recipient apheresis product is shipped to a cell culture and processing facility where a cell is processed.
4) After receipt of the recipient apheresis product, a recipient mononuclear cell is separated and collected by density gradient centrifugation at the cell culture processing facility and co-cultured with a thawed donor mononuclear cell and an inhibitory factor such as an anti-CD80 antibody and an anti-CD86 antibody or a CTLA4-Ig fusion protein.
5) A medium is exchanged about 7 days prior to administration. An intermediate product cultured for 7 days is collected and co-cultured with a thawed donor mononuclear cell and an inhibitory factor such as an anti-CD80 antibody and an anti-CD86 antibody or a CTLA4-Ig fusion protein.
6) On the day of administration, a cell-processed product is collected by density gradient centrifugation, then washed and filled into saline.
7) The resultant is shipped to a medical facility and administered to a recipient at the medical facility.

In a further aspect, the present disclosure provides a composition for treating a disease of a subject mediated by an immune response, the composition including an antigen-specific suppressor T cell selectively induced by mixing an inhibitory factor inhibiting interaction of IL-2 with an IL-2 receptor (IL-2R) and/or an inhibitory factor capable of inhibiting interaction of CD80 and/or CD86 with CD28, a cell derived from the subject, and an antigen derived from the subject or an antigen not derived from the subject or one containing the antigen.

In some embodiments, the disease may be selected from the group consisting of transplant immune rejection, an allergy, an autoimmune disease, a graft-versus-host disease, and immune rejection caused by transplantation of an iPS cell, or an ES cell, or a cell, tissue, or organ differentiated therefrom.

In some embodiments, the transplant immune rejection is characterized in that it is caused by transplantation of the kidney, the liver, the heart, the skin, the lung, the pancreas, the esophagus, the stomach, the small intestine, the large intestine, the nerve, blood, a blood cell including an immune system cell, the bone, the cartilage, a blood vessel, the cornea, the eyeball, or the bone marrow.

In embodiments in which the disease targeted by the present disclosure is transplant immune rejection, an anergy cell can be induced by mixing the inhibitory factor, a cell derived from a recipient, and an antigen derived from a donor or one containing the antigen derived from the donor. One containing the antigen derived from the donor may be a PBMC, a spleen cell, or a cell derived from the organ to be transplanted.

In an embodiment in which the disease targeted by the present disclosure is an allergy, an anergy cell can be induced by mixing the inhibitory factor, a cell derived from a subject, and an antigen that is not derived from the subject and that causes the allergy.

In an embodiment in which the disease targeted by the present disclosure is an autoimmune disease, an anergy cell can be induced by mixing the inhibitory factor, an antigen derived from a subject, and an antigen that is derived from the subject and that causes the autoimmune disease.

In an embodiment in which the disease targeted by the present disclosure is a graft-versus-host disease, an anergy cell can be induced by mixing the inhibitory factor, a PBMC or a spleen cell of a donor who provides a graft, a cell derived from a recipient or one containing the antigen. One containing the antigen derived from the recipient can be a PBMC, a spleen cell, or a cell in the proximity of a site to which an organ is transplanted or a cell derived therefrom.

In an embodiment in which a disease targeted by the present disclosure is immune rejection caused by transplantation of an iPS cell or an ES cell and a cell, tissue, or organ differentiated therefrom, an anergy cell can be induced by mixing the inhibitory factor, a cell derived from a subject, and a cell that is differentiated from the iPS cell or the ES cell and that is to be used for transplantation.

Examples of treatment of a disease according to the present disclosure will be described, but are not limited to the following.

### (Allergy and autoimmune disease)

In one aspect, the present disclosure provides a method for treating or preventing an allergy and/or an autoimmune disease using a medicament of the present disclosure, and a medicament, a composition, and a cell mixture to be used therefor. For the allergy and the autoimmune disease, a macrophage obtained from peripheral blood of a patient is conventionally differentiated into a dendritic cell (macrophage-derived dendritic cell) with a high antigen-presenting capacity. The cell is then irradiated (with γ-rays), allowed to present an antigen causing an excessive response in the allergy and the autoimmune disease, and co-cultured with a T cell population obtained from peripheral blood of the same patient in the presence of the above factor for 1 to 2 weeks, thereby obtaining an anergy cell specific for the antigen causing the allergy or the autoimmune disease. The anergy cell is administered to a patient to induce immune tolerance specific for an antigen causing the allergy or the autoimmune disease and used for prevention and treatment of the allergy or the autoimmune disease. The number of administrations may be multiple depending on whether the treatment is prophylactic or therapeutic and intensity of a symptom, etc.

### (Graft-versus-host disease)

In one aspect, the present disclosure provides a method for treating or preventing a graft-versus-host disease using a medicament of the present disclosure, and a medicament, a composition, and a cell mixture to be used therefor. For the graft-versus-host disease, in contrast to the treatment for the transplant immune rejection, a cell potentially causing the graft-versus-host disease such as a PBMC or a T cell of a donor that provides a graft is co-cultured with a PBMC or another cell derived from a host that has irradiated (with γ-rays) in the presence of the above factor for 1 to 2 weeks, thereby obtaining a host-specific anergy cell. The anergy cell is administered to the host to suppress a response to the host by the graft which causes the graft-versus-host disease (induce immune tolerance), thereby preventing and treating the graft-versus-host disease. The number of administrations may be multiple depending on whether the treatment is prophylactic or therapeutic, a size of a tissue to be transplanted, and intensity of a symptom, etc.

### (Application to treatment with iPS cell or ES cell)

In one aspect, the present disclosure provides a method for treating or preventing immune rejection or another side effect caused by transplantation of an iPS cell or an ES cell and a cell, tissue, or organ differentiated therefrom in prevention or treatment with the iPS cell or the ES cell using a medicament of the present disclosure; and a medicament, a composition, and a cell mixture to be used in the method. In application to the treatment with the iPS cell or the ES cell, immune rejection caused by transplantation of the iPS cell or the ES cell, and a cell, tissue, or organ differentiated therefrom is representatively exemplified as a therapeutic target.

The representative example will be described in detail below. For application to the treatment with the iPS cell or the ES cell, a cell that is differentiated from the iPS cell or the ES cell and that is to be used for transplantation, or a dendritic cell is irradiated (with γ-rays) and co-cultured with a PBMC or a T cell population of a patient to be transplanted in the presence of the above factor for 1 to 2 weeks, thereby obtaining an anergy cell specific for the cell differentiated from the iPS cell or the ES cell. The anergy cell is administered to a host to induce immune tolerance specific for a transplanted cell, tissue, and organ differentiated from the iPS cell or the ES cell, thereby preventing and treating rejection to them. The number of administrations may be multiple depending on whether the treatment is prophylactic or therapeutic, a size of a tissue to be transplanted, and intensity of a symptom.

### (Allergy)

In one aspect, the present disclosure provides a method for treating or preventing an allergy using a medicament of the present disclosure, and a medicament, a composition, and a cell mixture to be used therefor. The present inventors have demonstrated that a formulation containing an anergic cell antigen specifically induced by the above factor can elicit immune tolerance to an allergy. Thus, in an aspect of another aspect, the present disclosure provides a composition for treating or preventing an allergy in a subject, the composition including the above factor, a cell derived from the subject, and a cell with immune tolerance that is induced by mixing an antigen causing the allergy or one containing the antigen.

Examples of the antigen that causes the allergy include, but are not limited to, food, pollen, a drug, and metal, more specifically a mite antigen, an egg white antigen, a milk antigen, a wheat antigen, a peanut antigen, a soybean antigen, a buckwheat antigen, a sesame antigen, a rice antigen, a shellfish antigen, a kiwi antigen, an apple antigen, a banana antigen, a peach antigen, a tomato antigen, a tuna antigen, a salmon antigen, a mackerel antigen, a beef antigen, a chicken antigen, a pork antigen, a cat skin debris antigen, an insect antigen, a pollen antigen, a dog akin debris antigen, a fungal antigen, a bacterial antigen, a latex, a hapten, a metal, and the like.

### (Suppression or prevention of immune rejection caused by iPS cell, or the like)

In an aspect of one aspect, the present disclosure provides a method for suppressing or preventing immune rejection caused by an iPS cell or the like, and provides a medicament, a composition, and a cell mixture for the suppression or the prevention. The present inventors have demonstrated that a preparation containing an anergic cell induced by the above factor can elicit immune tolerance to immune rejection caused by an iPS cell or the like or a cell, tissue, or organ differentiated therefrom. Thus, in an aspect of another aspect, the present disclosure provides a composition for suppressing or preventing immune rejection caused by an iPS cell, or an ES cell, or a cell, tissue, or organ differentiated therefrom in a subject, the composition including a cell with immune tolerance that is induced by mixing the above factor, a cell derived from the subject, an antigen differentiated from the iPS cell or the ES cell or one containing the antigen.

Examples of the cell, tissue, or organ differentiated from the iPS cell or the ES cell include, but are not limited to, a nerve cell or tissue, a corneal cell or tissue, a cardiac muscle cell or tissue, a liver or tissue, a cartilage cell or tissue, a skin cell or tissue, a kidney or tissues, and the like. In a preferred embodiment, the cell, tissue, or organ differentiated from the iPS cell or the ES cell includes a nerve cell or tissue, a cardiac muscle cell or tissue, a cartilage cell or tissue, and a skin cell or tissue.

### (Notes)

As used herein, the term "or" is used when "at least one or more" of matters enumerated in a text can be employed. The same is true for "alternatively". When the phrase "within a range of two values" is specified herein, the range includes the two values themselves.

References cited herein such as scientific literatures, patents, and patent applications are incorporated herein by reference in its entirety as if specifically set forth herein.

The present disclosure has been described with reference to preferred embodiments for ease of understanding. Hereinafter, the present disclosure will be described with reference to Examples, but the above description and Examples below are provided for illustrative purposes only and are not intended to limit the present disclosure. Accordingly, the scope of the present disclosure is not limited to embodiments or Examples specifically described herein, but is limited only by claims.

### [Example]

The present disclosure will be specifically described with reference to Examples. However, the present disclosure is not limited to the Examples. Note that, all references cited throughout this specification are hereby incorporated by reference in their entirety.

### (Antibody)

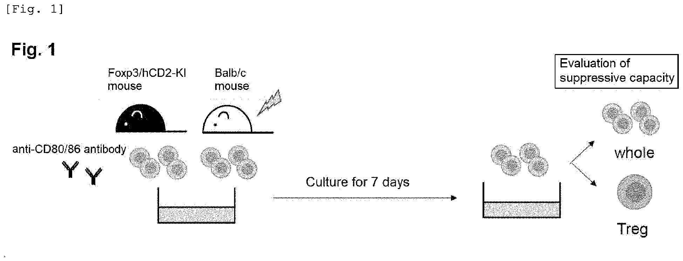

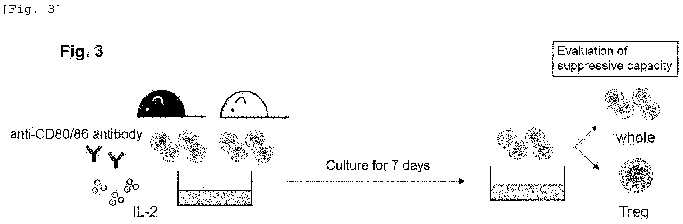

Antibodies used in Examples below are as follows: anti-CD80 monoclonal antibody (mAb): clone RM80 anti-CD86 monoclonal antibody (mAb): clone GL-1 anti-IL-2 monoclonal antibody (mAb): InVivoMAb anti-mouse IL-2; manufacturer: Bio X Cell; model number: BE0043 (Example 1: Effect of IL-2 addition on allospecific suppression of Treg). In this example, it was confirmed that addition of IL-2 in the presence of an anti-CD80/86 antibody in a mixed lymphocyte reaction (MLR) attenuated an antigen-specific suppressive capacity of a Treg. An overview of Example 1 is shown in FIGs. 1 and 3.

### (Method)

1. Spleen cells were collected from Foxp3/hCD2-KI C57BL6 mice (hCD2-KI mice) and prepared to 4 x 10⁶ cells/mL.
2. Spleen cells were taken from Balb/c mice, irradiated with X-rays at 30 Gy, prepared to a concentration of 4 x 10⁶ cells/mL, and mixed at a 1:1 ratio with the spleen cells from the hCD2-KI mice.
3. The anti-CD80 mAb + the anti-CD86 mAb (final concentration: 10 µg/mL each) and, if necessary, recombinant IL-2 (final concentration: 25 U/mL) were added thereto, seeded in a 10 cm dish, and cultured in a 5% CO² incubator at 37°C for 7 days.
4. Seven days after the culture, the cells were collected and Tregs were separated using magnetic beads if necessary.
5. Spleen cells from B6 mice, LPS-stimulated spleen cells from Balb/c mice, or LPS-stimulated spleen cells from CBA/NSlc mice, and whole cells cultured in 3. (whole cells) or separated Tregs were prepared to the concentrations shown in Table 1 and seeded into a U-bottom 96-well plate.
6. The resultant was cultured in a 5% CO² incubator at 37°C for 3 days.

**[Table 1]**

| Cell | | Cell concentration | I Seeding ratio | | | | |
|---|---|---|---|---|---|---|---|
| | | | 1:0 | 1:1/8 | 1:1/16 | 1:1/32 | (-) |
| Resp | hCD2-KI | 2x10⁶cells/mL | 100µL | 100µL | 100µL | 100µL | 100µL |
| Stim | LPS-Balb/c | 2x10⁶cells/mL | 100µL | 100µL | 100µL | 100µL | 0µL |
| | LPS-CBA (rad+) | | | | | | |
| Culture cells | Whole/Treg | 0.5x10⁶cells/mL | 0µL | 50µL | 25µL | 12.5µL | 0µL |
| Medium | | | 50µL | 0µL | 25µL | 37.5µL | 150µL |
| Total | | | 250µL | 250µL | 250µL | 250µL | 250µL |

7. On the third day of culture, a culture supernatant was collected and cryopreserved at -20°C.
8. The thus-collected culture supernatant was measured for IFN-γ by ELISA.

### (Evaluation of Treg division by CFSE)

1. Spleen cells were collected from Foxp3/hCD2-KI C57BL6 mice (hCD2-KI mice) and prepared to 2 x 10⁶ cells/mL.
2. Spleen cells were taken from Balb/c mice, irradiated with X-rays at 30 Gy, prepared to a concentration of 2 x 10⁶ cells/mL, and mixed at a 1:1 ratio with the spleen cells from the hCD2-KI mice.
3. The anti-CD80 mAb + the anti-CD86 mAb (final concentration: 10 µg/mL each) or the anti-IL-2 mAb (final concentration: 10 µg/mL) and, if necessary, recombinant IL-2 (final concentration: 25 U/mL) were added thereto, seeded in 200 µL in each well of a U-bottom 96-well plate, and cultured in a 5% CO² incubator at 37°C for 4 days.
4. Four days after the culture, cells were collected, dead cells were stained with zombie Violet, followed by a PerCP/Cy5.5-CD3 antibody and an APC-hCD2 antibody. The percentage of CD3+hCD2+CFSE- cells was measured with FACSLyric.

### (Results)

The results are shown in Table 2. Proliferation of Tregs was suppressed when cultured in the presence of the anti-CD80mAb + the anti-CD86mAb. Production of IFN-γ was suppressed when LPS-Balb/c and the whole cells were used as stimulators, and the production of IFN-γ was more strongly suppressed, when the isolated Tregs were used. Thus, the anti-CD80 mAb + the anti-CD86 mAb is believed to promote proliferation of antigen-specific induced suppressor T cells.

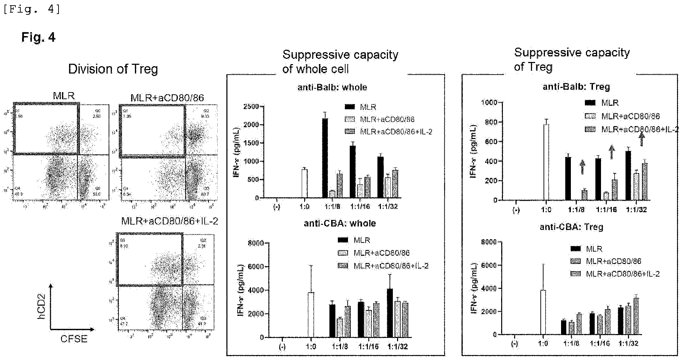

On the other hand, when IL-2 was further added, more Tregs proliferated than when no IL-2 was added. When the LPS-Balb/c as well as the whole cells and the Tregs cultured in the presence of IL-2 were used as stimulators, production of IFN-γ was increased and its immune response suppressive capacity was attenuated (FIG. 4). Thus, it is believed that IL-2 also promotes proliferation of non-antigen-specific Tregs, resulting in loss of an antigen-specific suppressive capacity. When LPS-stimulated spleen cells from CBA/Nslc mice were used as a stimulator, immune responses were observed in both groups.

### (Example 2: Evaluation of allospecific suppressive capacity of Tregs in MLR with anti-IL-2 antibody)

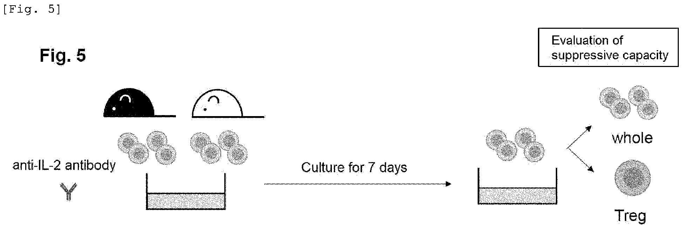

In this example, it was confirmed that addition of an anti-IL-2 antibody in MLR induced an antigen-specific suppressive activity in Tregs, similar to that induced by addition of an anti-CD80/86 antibody. An overview of Example 2 is shown in FIG. 5.

### (Method)

1. Spleen cells were collected from Foxp3/hCD2-KI C57BL6 mice (hCD2-KI mice) and prepared to 4 x 10⁶ cells/mL.
2. Spleen cells were taken from Balb/c mice, irradiated with X-rays at 30 Gy, prepared to a concentration of 4 x 10⁶ cells/mL, and mixed at a 1:1 ratio with the spleen cells from the hCD2-KI mice.
3. The anti-CD80mAb + the anti-CD86mAb (final concentration: 10 µg/mL each) or anti-IL-2 mAb (final concentration: 10 µg/mL) was added thereto, seeded into a U-bottom 96-well plate, and cultured in a 5% CO² incubator at 37°C for 7 days.
4. Seven days after the culture, the cells were collected and Tregs were separated using magnetic beads if necessary.
5. Spleen cells from B6 mice, LPS-stimulated spleen cells from Balb/c mice, or LPS-stimulated spleen cells from CBA/NSlc mice, and whole cells cultured in 3. (whole cells) or separated Tregs were prepared to the concentrations shown in Table 2 and seeded into a U-bottom 96-well plate.
6. The resultant was cultured in a 5% CO² incubator at 37°C for 3 days.

**[Table 2]**

| **Cell** | | **Cell concentration** | **Seeding ratio** | | | | |
|---|---|---|---|---|---|---|---|
| | | | 1:0 | 1:1/8 | 1:1/16 | 1:1/32 | (-) |
| Resp | hCD2-KI | 2x10⁶cells/mL | 100µL | 100µL | 100µL | 100µL | 100µL |
| Stim | LPS-Balb/c | 2x10⁶cells/mL | 100µL | 100µL | 100µL | 100µL | 0µL |
| | LPS-CBA (rad+) | | | | | | |
| Culture cells | Whole/Treg | 0.5x10⁶cells/mL | 0µL | 50µL | 25µL | 12.5µL | 0µL |
| Medium | | | 50µL | 0µL | 25µL | 37.5µL | 150µL |
| Total | | | 250µL | 250µL | 250µL | 250µL | 250µL |

7. On the third day of culture, a culture supernatant was collected and cryopreserved at -20°C.
8. The thus-collected culture supernatant was measured for IFN-γ by ELISA.

### (Results)

The results are shown in Table 6. It was observed that addition of the anti-IL-2 antibody induced a suppressive capacity in the whole cells, although not so much as when the anti-CD80/86 antibody was added. On the other hand, an antigen-specific suppressive capacity was induced in the Tregs comparable to that induced by addition of the anti-CD80/86 antibody. When the LPS-stimulated spleen cells from CBA/Nslc mice were used as the stimulator, immune responses were observed in both groups.

### (Discussion)

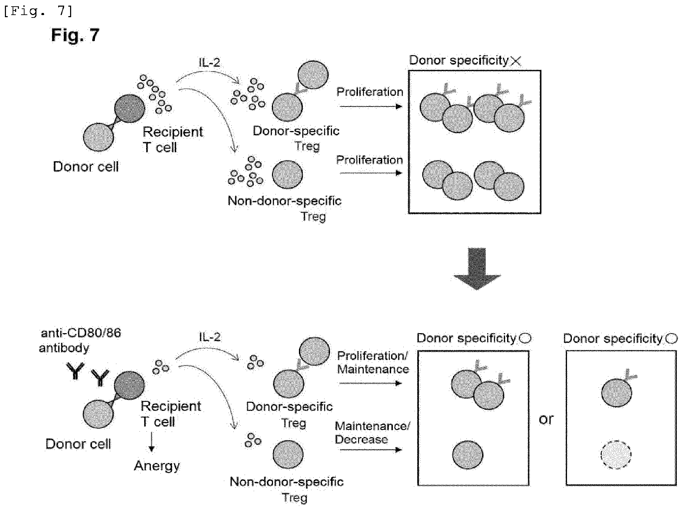

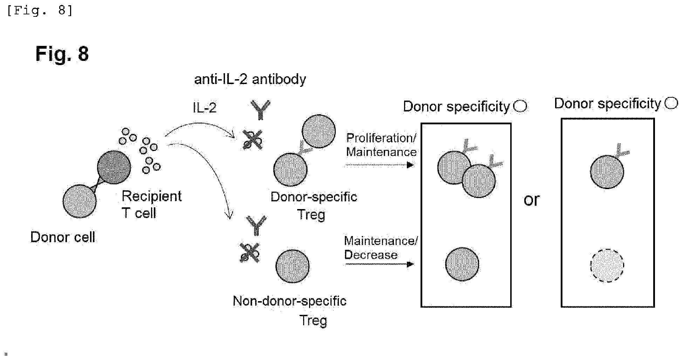

The process of proliferating Tregs with IL-2 is present in almost all Treg therapies. However, the IL-2 induces proliferation of polyclonal (bulk) Tregs, which may attenuate the effect (FIG. 7). Addition of the anti-CD80/86 antibody or the anti-IL-2 antibody suppresses proliferation of the polyclonal (bulk) Tregs by suppressing production of a growth factor IL-2 produced during MLR. On the other hand, donor-responsive Tregs selectively survive depending on stimulation from donor-responsive TCRs and a small amount of IL-2. As a result, the whole cells and the Tregs exhibit a donor-specific suppressive capacity in cells induced by MLR with the anti-CD80/86 antibody or the anti-IL-2 antibody (FIGs. 7 and 8).

### (Example 3: Effect of IL-2 in culture)

In this example, the effect of IL-2 on induction of an antigen-specific induced suppressor T cell using an anti-CD80/86 antibody and/or an anti-IL-2 antibody was confirmed. The method was the same as in Examples 1 and 2. The concentration of IL-2 in a culture solution on Days 1 to 5 of culture was measured by ELISA.

### (Results)

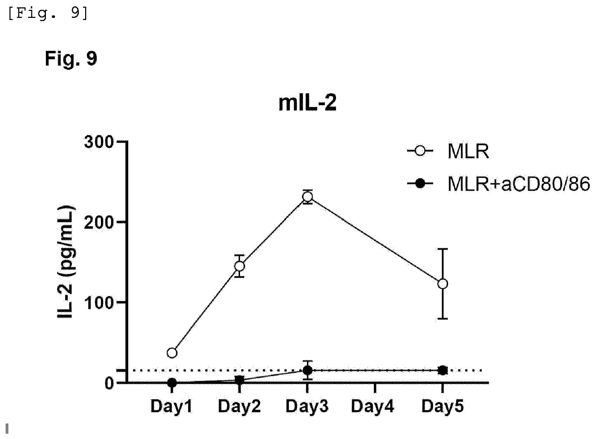

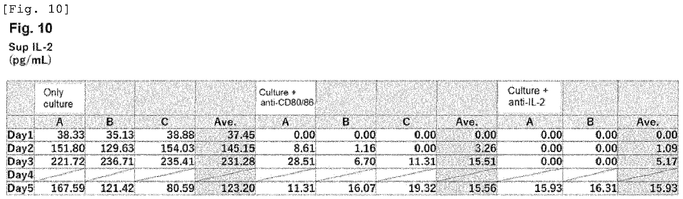

FIGs. 9 and 10 show that when cultured in the presence of the anti-CD80/86 antibody or the anti-IL-2 antibody, IL-2 had a concentration of 20 pg/mL or less on Day 5. It is believed that the anti-CD80/86 antibody inhibited a CD28 co-stimulatory signal, resulting in reduced T cell activation and suppression of IL-2 production. On the other hand, when cultured without the anti-CD80/86 antibody or the anti-IL-2 antibody, IL-2 had a concentration above 100 pg/mL as early as on the second day of culture.

### (Example 4: Change in percentage of CD4⁺ T cells by anti-CD80/86 antibody or anti-IL-2 antibody)

In this example, a change in percentage of CD4⁺ T cells by the anti-CD80/86 antibody and/or the anti-IL-2 antibody was determined. The method was the same as in Examples 1 and 2.

### (Results)

The results are shown in Table 11. Addition of the anti-CD80/86 antibody increased the percentage of CD4⁺ T cells and Tregs (CD4⁺FOXP3⁺ T cells) relative to total cells compared to "no addition in culture" and also increased the percentage of the Tregs relative to the CD4⁺ T cells. Addition of an anti-IL-2 neutralizing antibody did not increase the percentage of CD4⁺ T cells and Tregs (CD4⁺FOXP3⁺ T cells) relative to total cells compared to "no addition in culture" and also increased the percentage of the Tregs relative to the CD4⁺ T cells.

### (Example 5: Change in suppressive capacity by anti-CD80/86 antibody or anti-IL-2 antibody)

In this example, a change in suppressive capacity by the anti-CD80/86 antibody and/or the anti-IL-2 antibody was verified.

### (Results)

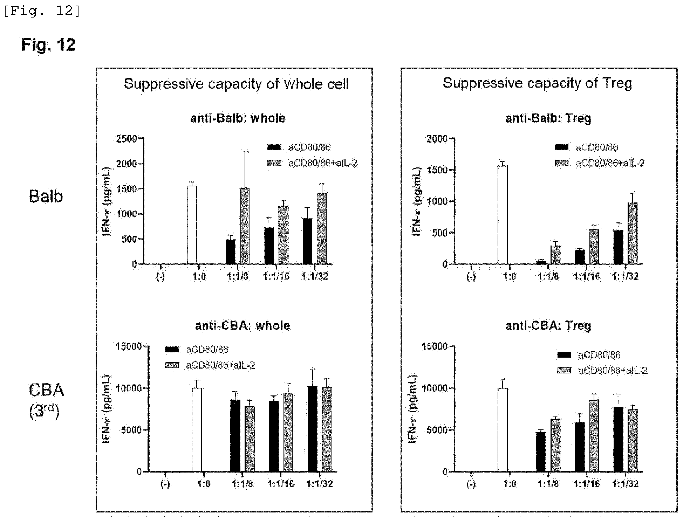

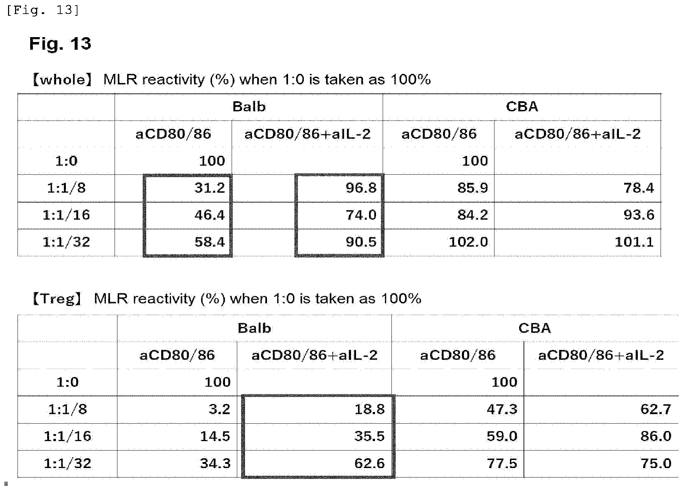

Complete blockade of IL-2 with both the anti-CD80/86 antibody and the anti-IL-2 antibody resulted in a suppressive capacity against Balb stimulation in the isolated Tregs and almost no suppression in the whole cells. When cultured with only the anti-CD80/86 antibody, a stronger suppressive capacity against Balb stimulation was shown. These results indicate that the suppressive capacity of the suppressor T cell is IL-2-dependent. The isolated Tregs also showed a slight suppressive capacity against CBA, but almost no suppressive capacities in the whole cells (FIGs. 12 and 13). Thus, the suppressive capacity of the whole cell is highly antigenic and highly IL-2-dependent. Non-antigen-specific suppression may be due to the effect of an inhibitory cytokine such as TGF-β.

(Example 6: Evaluation of suppression of IL-2 production by anti-CD80/86 antibody or anti-IL-2 neutralizing antibody)

In this example, human cells are used to confirm that production of IL-2 is suppressed by the anti-CD80/86 antibody or the anti-IL-2 neutralizing antibody.

### (Material)

· Responder-PBMC 4 x 10⁶/mL 100 µL
· X ray-irradiated Stimulator-PBMC 4 x 10⁶/mL 100 µL
· Anti-CD80/86 antibody Final concentration: 10 µg/mL
· Anti-IL-2 neutralizing antibody Final concentration: 10 µg/mL

### (Method)

1. Peripheral blood lymphocytes (PBMCs) are collected from three healthy subjects.
2. The cells are combined into 3 pairs and the cells serving as the Stimulator are irradiated with X-rays at 30 Gy.
3. The cells are seeded in a 96-well plate under the following conditions and cultured at 37°C, 5% CO2.
   · Responder PBMCs 4 x 10⁶/mL 100 µL
   · X ray-irradiated Stimulator-PBMC 4 x 10⁶/mL 100 µL
   · Anti-CD80/86 antibody Final concentration: 10 µg/mL
   · Anti-IL-2 neutralizing antibody Final concentration: 10 µg/mL
4. On the first, second, third, and fifth days of the culture, a culture supernatant is collected and cryopreserved at -20°C until IL-2 is measured by ELISA.
5. Human IL-2 is measured by ELISA.

### (Results)

When cultured in the presence of the anti-CD80/86 antibody or the anti-IL-2 antibody, a concentration of IL-2 in the culture supernatant is expected to be lower than in a no-addition group.

### (Example 7: Evaluation of effect of regulation of IL-2 concentration in culture solution on donor-specific suppressive capacity of Treg)

### (Method)

1. Spleen cells are collected from Foxp3/hCD2-KI C57BL6 mice (hCD2-KI mice) and prepared to 4 x 10⁶ cells/mL.
2. Spleen cells are taken from Balb/c mice, irradiated with X-rays at 30 Gy, prepared to a concentration of 4 x 10⁶ cells/mL, and mixed at a 1:1 ratio with the spleen cells from the hCD2-KI mice.
3. Anti-IL-2 mAb (final concentration: 10 µg/mL) was added thereto, seeded in a 10 cm dish, and cultured in a 5% CO2 incubator at 37°C.
4. On the second and fourth days of culture, IL-2 was added to a concentration of 15 pg/mL.
5. Seven days after the culture, the cells are collected and Tregs are separated using magnetic beads if necessary.
6. Spleen cells from B6 mice, LPS-stimulated spleen cells from Balb/c mice, or LPS-stimulated spleen cells from CBA/NSlc mice, and the whole cells cultured in 5. (whole cells) or the separated Tregs are prepared to the concentrations shown in Table 1 and seeded into a U-bottom 96-well plate.
7. The resultant was cultured in a 5% CO2 incubator at 37°C for 3 days.

Specifically, the following experiments were performed as follows.

### (Measurement of IL-2)

Two types of commercially available human peripheral blood mononuclear cells (LONZA CC-2702) were used as donor and recipient cells. The donor cells (Batch# 22TL290862) were suspended in 15 mL of an RPMI medium (FUJIFILM Wako Pure Chemical Corporation) containing 10% FCS (SELBORNE) and then irradiated at 25 Gy. Next, an anti-CD80 antibody (manufactured by JUNTEN BIO Co., Ltd.) was added to the donor cells and the recipient cells (Batch# 23TL043676) at a final concentration of 5 mg/L, respectively, and then the resultant was left to stand in ice for 30 minutes. The donor cells and the recipient cells were then collected by centrifugation, and a CD86 antibody (manufactured by JUNTEN BIO Co., Ltd.) was added to the donor cells at a final concentration of 5 mg/L. Next, the donor cells and the recipient cells were prepared to a final concentration of 5.0 x 10^7 cells/20 mL, respectively, in two T flasks (Thermo scientific 25 cm²) containing 20 mL of a 10% FCS-containing RPMI medium. An anti-CD80 antibody (manufactured by JUNTEN BIO Co., Ltd.) and an anti-CD86 antibody (manufactured by JUNTEN BIO Co., Ltd.) were added to one T flask at a final concentration of 5 mg/L, respectively, and the other flask was used as a negative control without adding antibodies. The two T flasks were left to stand in a 5% CO₂ incubator set at 37°C. Culture supernatants were collected before the start of culture and on Days 1, 2, 3, 4, and 7 after the start of culture and measured for a concentration of IL-2 by the Cytometric Bead Array (CBA) method, and the results are shown in Table 3. Addition of the anti-CD80 antibody and the anti-CD86 antibody resulted in a marked decrease in an IL-2 level from Day 4 of 7 days of culture onward compared to the negative control.

**[Table 3]**

| Table 3 Decrease of IL-2 concentration in culture supernatant during production of JB-101 | | | | | | |
|---|---|---|---|---|---|---|
| Days of culture | 0 | 1 | 2 | 3 | 4 | 7 |
| Negative control | 0.0 | 114.0 | 18.7 | 36.5 | 524.8 | 115.9 |
| Addition of antibody | 0.0 | 30.8 | 70.5 | 57.0 | 19.8 | 0.6 |

### (Evaluation of MLR suppression)

The cells cultured for 7 days as described above were added to a co-culture system of donor cells (Batch# 22TL290862: 2.0 x 10^5 cells/well) and recipient cells (Batch# 23TL043676: 2.0 x 10^5 cells/well) irradiated at 25 Gy and cultured in a 96-well plate (CORNING). Five days later, concentrations of IFN-γ and granzyme B in a culture supernatant were measured by the CBA method. The results are shown in Table 4. Addition of the cells cultured in the presence of the anti-CD80 antibody and the anti-CD86 antibody resulted in a cell concentration-dependent decrease in concentrations of the IFN-γ and the granzyme B, confirming that MLR was suppressed.

**[Table 4]**

| Table 4 MLR (IFN-γ production) suppressive effect of JB-101 product | | | | | | | |
|---|---|---|---|---|---|---|---|
| Added cell/MLR cell (ratio) | | 0 | 1/2 | 1/4 | 1/8 | 1/16 | |
| Negative control / Administration of antibody / | | 503.7 | 205.1 | 876.9 | 385.5 | 856.1 | pg/mL |
| | | 357.5 | 65.8 | 154.3 | 184.7 | 375.5 | pg/mL |

### (Results)

An allospecific suppressive capacity of the Tregs was enhanced when IL-2 was added during culture and adjusted to the optimum concentration.

### (Notes)

Although the present disclosure has been illustrated with reference to preferred embodiments of the present disclosure, it is understood that the scope of the present disclosure should be construed only by the claims. It is to be understood that patents, patent applications, and other references cited herein should be incorporated herein by reference in its entirety as if the contents themselves are specifically set forth herein. The present disclosure claims priority to Japanese Patent Application No. 2023-012172 filed on January 30, 2023 with the Japan Patent Office and it is understood that the content thereof is cited herein in its entirety as constituting the specification.

### [Industrial Applicability]

A technology available in an industry (pharmaceutical industry) relating to formulation and the like is provided.

## Claims

1. A method for selectively inducing an antigen-specific induced suppressor T cell from a cell derived from a subject, the method comprising
mixing a factor selectively inducing an antigen-specific induced suppressor T cell, a cell derived from the subject, and an antigen derived from the subject or an antigen not derived from the subject or one containing the antigen not derived from the subject.

2. The method according to claim 1, wherein the factor selectively inducing the antigen-specific induced suppressor T cell is a regulatory factor capable of regulating production of IL-2 or a regulatory factor capable of regulating a function of IL-2 produced.

3. The method according to claim 1, wherein the factor selectively inducing the antigen-specific induced suppressor T cell is an inhibitory factor capable of inhibiting production of IL-2 or an inhibitory factor capable of inhibiting a function of IL-2 produced.

4. The method according to any one of claims 1 to 3, wherein the factor selectively inducing the antigen-specific induced suppressor T cell is an inhibitory factor capable of inhibiting interaction of IL-2 with an IL-2 receptor (IL-2R) and/or an inhibitory factor capable of inhibiting interaction of CD80 and/or CD86 with CD28.

5. The method according to any one of claims 1 to 4, wherein the factor selectively inducing the antigen-specific induced suppressor T cell is the inhibitory factor capable of inhibiting interaction of IL-2 with the IL-2R, the inhibitory factor being selected from an anti-IL-2 antibody, an anti-IL-2R antibody, an anti-IL-2Rα (CD25) antibody, an anti-IL-2Rβ (CD122) antibody, or an antigen-binding fragment thereof.

6. The method according to any one of claims 1 to 5, wherein the factor selectively inducing the antigen-specific induced suppressor T cell is the inhibitory factor capable of inhibiting interaction of CD80 and/or CD86 with CD28, the inhibitory factor being selected from the group consisting of an anti-CD80 antibody, an anti-CD86 antibody, a bispecific antibody against CD80 and CD86, an anti-CD28 antibody, or an antigen-binding fragment thereof, a CTLA4-Ig fusion protein, and a CD28-Ig fusion protein.

7. A method for producing an induced suppressor T cell from a cell derived from a subject, the method comprising
mixing a regulatory factor capable of regulating production of IL-2 or a regulatory factor capable of regulating a function of IL-2 produced, a cell derived from the subject, and an antigen derived from the subject or an antigen not derived from the subject or one containing the antigen.

8. The method according to claim 7, wherein the regulatory factor capable of regulating production of IL-2 is selected from an inhibitory factor capable of inhibiting interaction of CD80 and/or CD86 with CD28, and a small molecular compound inhibiting production of IL-2.

9. The method according to claim 7, wherein the regulatory factor capable of regulating the function of IL-2 produced is selected from an inhibitory factor capable of inhibiting interaction of IL-2 with an IL-2 receptor (IL-2R) and a small molecular compound inhibiting an action of IL-2.

10. The method according to claim 8, wherein the inhibitory factor capable of inhibiting interaction of CD80 and/or CD86 with CD28 is selected from the group consisting of an anti-CD80 antibody, an anti-CD86 antibody, a bispecific antibody against CD80 and CD86, an anti-CD28 antibody, or an antigen-binding fragment thereof, a CTLA4-Ig fusion protein, and a CD28-Ig fusion protein.

11. The method according to claim 9, wherein the inhibitory factor capable of inhibiting interaction of IL-2 with the IL-2 receptor (IL-2R) is selected from an anti-IL-2 antibody, an anti-IL-2R antibody, an anti-IL-2Rα (CD25) antibody, an anti-IL-2Rβ (CD122) antibody, or an antigen-binding fragment thereof.

12. A composition for selectively inducing an antigen-specific induced suppressor T cell from a cell derived from a subject, the composition comprising
a regulatory factor capable of regulating production of IL-2 or a regulatory factor capable of regulating a function of IL-2 produced,
the composition being brought into contact with a cell derived from the subject and an antigen derived from the subject or an antigen not derived from the subject or one containing the antigen.

13. A composition for treating a disease of a subject mediated by an immune response, the composition comprising
an antigen-specific induced suppressor T cell selectively induced by mixing a regulatory factor capable of regulating production of IL-2 or a regulatory factor capable of regulating a function of IL-2 produced, a cell derived from the subject, and an antigen derived from the subject or an antigen not derived from the subject or one containing the antigen.

14. The composition according to claim 13, wherein the disease is selected from the group consisting of transplant immune rejection, an allergy, an autoimmune disease, a graft-versus-host disease, and immune rejection caused by transplantation of an iPS cell, or an ES cell, or a cell, tissue, or organ differentiated therefrom.
